# EUROPEAN PATENT SPECIFICATION

(11) **EP 3 596 095 B1**
(45) Date of publication and mention of the grant of the patent: **19.08.2026**
(21) Application number: 18764563.5
(22) Date of filing: 01.03.2018
(51) Int. Cl.: C07H 1/00, C07H 3/06, A61K 31/702, A23L 33/00, A23L 33/125

(54) **PROCESS FOR CRYSTALLIZING 2'-FUCOSYLLACTOSE AND RELATED COMPOSITIONS**
VERFAHREN ZUR KRISTALLISIERUNG VON 2'-FUCOSYLLACTOSE UND ZUGEHÖRIGE ZUSAMMENSETZUNGEN
PROCÉDÉ DE CRISTALLISATION DU 2'-FUCOSYLLACTOSE ET COMPOSITIONS ASSOCIÉES

(30) Priority: 06.03.2017 US 201762467571 P; 21.04.2017 EP 17167564; 19.02.2018 US 201862632180 P
(43) Date of publication of application: 22.01.2020
(73) Proprietor: Inbiose N.V., 9052 Zwijnaarde (BE)
(72) Inventor: NURMI, Juha, 02400 Kirkkonummi (FI); BACKMAN, Pentti, 08500 Lohja (FI); MARTIKAINEN, Heidi, 01300 Vantaa (FI); SIITONEN, Jani, 00200 Helskinki (FI)
(74) Representative: Saelens, Claire
(86) International application number: PCT/US2018/020431
(87) International publication number: WO 2018/164937

(56) References cited:
- WO-A1-2011/150939
- WO-A1-2013/185780
- WO-A1-2014/100191
- WO-A1-2015/188834
- WO-A1-2016/095924
- WO-A2-2010/070616
- WO-A2-2014/009921
- GB-B- 2 155 934
- US-A1- 2013 165 406
- US-B2- 7 015 238

## Description

### FIELD

This specification relates to a process for crystallizing an oligosaccharide, particularly a human milk oligosaccharide, and, more particularly, 2'-fucosyllactose ("2'-FL"). This specification also relates to compositions (e.g., crystalline products) produced using such a process.

### BACKGROUND

Human milk oligosaccharides are important for nutrition and therapeutics. One of the most important human milk oligosaccharides is 2'-fucosyllactose (also known as 2'-O-fucosyllactose or "2'-FL"). This oligosaccharide is the most abundant oligosaccharide found in human breast milk, and is believed to have several beneficial biological roles, including prebiotic, antibacterial, antiviral, immune system enhancing and brain development enhancing effects. Such benefits make 2'-FL a potentially attractive ingredient to be included in food, dietary supplements and medicines, and particularly infant formula. However, development of economically feasible processes for large scale production of 2'-FL continues to be a challenge.

Many recent approaches for synthesis of 2'-FL involves microbial fermentation processes, which produce 2'-FL from lactose. While there has been success with respect to this approach, such processes generally produce a complex product mixture, which often includes other ingredients such as lactose, oligosaccharides besides the desired 2'-FL (*e.g.,* difucosyllactose ("DiFL")), monosaccharides, amino acids, polypeptides, proteins, monovalent and divalent salts, organic acids, nucleic acids, etc. Thus, there continues to be a need for effective, reliable and economically feasible downstream purification processes that provide a useable 2'-FL product.

One technique for purification of 2'-FL is crystallization. However, commonly-used cooling crystallization techniques generally fail to work with 2'-FL due to its unique crystallization properties. While saturated water syrups of 2'-FL exhibit low viscosity at low temperatures (which normally suggests crystallization will be straight forward), even highly supersaturated aqueous 2'-FL syrups are generally stable at low temperatures.

To overcome this problem, some in the art have reported techniques involving the addition of organic solvents to force crystallization. *See, e.g.,* Kuhn, R., et al., Chemische Berichte, vol. 88(8), pp. 1135-1146 (1955); Kuhn, R., et al., Chemische Berichte, vol. 89(11), p. 2513 (1956); WO2011/150939; WO2015/188834; and WO201695924. There are, however, many potential disadvantages of using an organic solvent to crystallize 2'-FL. These include, for example, the cost of the organic solvent itself. Organic solvents also can create harmful emissions, waste and/or pollution. Special equipment is often required to recover the solvent after the crystallization. In some instances, there are solvent losses associated with such solvent recovery. For some solvents, special equipment is also needed due to explosion or other safety reasons. And use of solvents also can be problem if residual solvent remains in the crystalline product. Even trace levels of some organic solvents may have an adverse effect on taste, odor, color and/or food safety.
WO 2014/009921 A2 and WO 2015/188834 Al also relate to production methods of polymorphs of 2'-FL.

Accordingly, a need continues to exist for an effective, reliable and economically feasible downstream process for purifying 2'-FL, particularly an aqueous crystallization that avoids the need for an organic solvent.

### SUMMARY

Briefly, this specification generally discloses a process for crystallizing 2'-FL. The subject matter of the present invention is set out in the claims.

In particular, this specification discloses, in part, a process for making crystalline 2'-FL from an aqueous starting solution comprising 2'-FL and at least one other carbohydrate.

The process comprises concentrating the starting solution to a supersaturated state with respect to 2'-FL, and then precipitating a 2'-FL crystal from the supersaturated solution while subjecting the supersaturated solution to a temperature of greater than 60°C. In this process, the supersaturated solution comprises no greater than 1% (by weight) organic solvent during the precipitation of the 2'-FL crystal.

In some embodiments, the process comprises concentrating the starting solution to a supersaturated state with respect to 2'-fucosyllactose, and then precipitating a 2'-fucosyllactose crystal from the supersaturated solution while subjecting the supersaturated solution to a temperature of at least 40°C. In this process, the starting solution has a dry solids content with a 2'-fucosyllactose concentration of from 70 to 95% (wherein the percentage corresponds to a normalized peak area concentration obtained using high performance liquid chromatography). In addition, the supersaturated solution comprises no greater than 1% (by weight) organic solvent during the precipitation of the 2'-fucosyllactose crystal.

In some embodiments, the process comprises concentrating the starting solution to a supersaturated state with respect to 2'-fucosyllactose, and then precipitating a 2'-fucosyllactose crystal from the supersaturated solution while subjecting the supersaturated solution to a temperature of at least 40°C. In this process, the starting solution has a dry solids content with a 2'-fucosyllactose concentration of less than about 98% (wherein the percentage corresponds to a normalized peak area concentration obtained using high performance liquid chromatography). In addition, the supersaturated solution comprises no greater than 1% (by weight) organic solvent during the precipitation of the 2'-fucosyllactose crystal.

In some embodiments, the process comprises concentrating the starting solution to a supersaturated state with respect to 2'-fucosyllactose, and then precipitating a 2'-fucosyllactose crystal from the supersaturated solution while subjecting the supersaturated solution to a temperature of at least 40°C. In this process, the starting solution has a dry solids content with a 2'-fucosyllactose concentration of less than about 95% (wherein the percentage corresponds to a normalized peak area concentration obtained using high performance liquid chromatography). In addition, the supersaturated solution comprises no greater than 1% (by weight) organic solvent during the precipitation of the 2'-fucosyllactose crystal.

The process comprises concentrating the starting solution to a supersaturated state with respect to 2'-fucosyllactose, and then precipitating a 2'-fucosyllactose crystal from the supersaturated solution while subjecting the supersaturated solution to a temperature of at least 60°C. In this process, the supersaturated solution comprises no greater than 1% (by weight) organic solvent during the precipitation of the 2'-fucosyllactose crystal. In addition, the crystalline 2'-fucosyllactose product has a melting point of from about 230 to about 239°C (as determined with a 1°C/min heating rating using the European Pharmacopoeia capillary melting point method).

In some embodiments, the process comprises concentrating the starting solution to a supersaturated state with respect to 2'-fucosyllactose, and then precipitating a 2'-fucosyllactose crystal from the supersaturated solution while subjecting the supersaturated solution to a temperature of at least 40°C. In this process, the supersaturated solution comprises no greater than 1% (by weight) organic solvent during the precipitation of the 2'-fucosyllactose crystal. In addition, the crystalline 2'-fucosyllactose product exhibits an X-ray powder diffraction reflection, based on a measurement using CuKa radiation, at 16.98±0.20, 13.65±0.20 and 18.32±0.20 2Θ angles.

This specification also discloses, in part, a process for making amorphous 2'-FL. The process comprises making crystalline 2'-FL according to the crystallization process described above (or elsewhere in this specification), dissolving the crystalline 2'-FL in a solvent (e.g., water) to form a purified 2'-FL solution, and precipitating amorphous 2'-FL from the purified 2'-FL solution.

This specification also discloses, in part, a processes for making a food, dietary supplement or medicine.

In some embodiments, the process for making the food, dietary supplement or medicine comprises making crystalline 2'-FL according to the crystallization process described above (or elsewhere in this specification), and then mixing the crystalline 2'-FL with one or more ingredients suitable for the food, dietary supplement or medicine.

In some embodiments, the process for making the food, dietary supplement or medicine comprises making crystalline 2'-FL according to the crystallization process described above (or elsewhere in this specification), dissolving the crystalline 2'-FL in a solvent (e.g., water), and mixing the dissolved 2'-FL with one or more ingredients suitable for the food, dietary supplement or medicine.

In some embodiments, the process for making the food, dietary supplement or medicine comprises making amorphous 2'-FL as described above (or elsewhere in this specification), and then mixing the amorphous 2'-FL with one or more ingredients suitable for the food, dietary supplement or medicine.

In some embodiments, the process for making the food, dietary supplement or medicine comprises making amorphous 2'-FL as described above (or elsewhere in this specification), dissolving the amorphous 2'-FL in a solvent (*e.g*., water), and mixing the dissolved 2'-FL with one or more ingredients suitable for the food, dietary supplement or medicine.

This specification also discloses, in part, processes for making infant formula.

In some embodiments, the process for making infant formula comprises making crystalline 2'-FL according to the crystallization process described above (or elsewhere in this specification), and then mixing the crystalline 2'-FL with one or more infant formula ingredients.

In some embodiments, the process for making infant formula comprises making crystalline 2'-FL according to the crystallization process described above (or elsewhere in this specification), dissolving the crystalline 2'-FL in a solvent (*e.g*., water), and mixing the dissolved 2'-FL with one or more infant formula ingredients.

In some embodiments, the process for making an infant formula comprises making amorphous 2'-FL as described above (or elsewhere in this specification), and then mixing the amorphous 2'-FL with one or more infant formula ingredients.

In some embodiments, the process for making an infant formula comprises making amorphous 2'-FL as described above (or elsewhere in this specification), dissolving the amorphous 2'-FL in a solvent (*e.g*., water), and mixing the dissolved 2'-FL with one or more infant formula ingredients.

This specification also discloses, in part, a crystalline 2'-FL product obtained from a crystallization process described above (or elsewhere in this specification).

This specification also discloses, in part, amorphous 2'-FL obtained from a process described above (or elsewhere in this specification) for making amorphous 2'-FL.

This specification also discloses, in part, a food, dietary supplement or medicine prepared by a process for making a food, dietary supplement or medicine described above (or elsewhere in this specification).

This specification also discloses, in part, an infant formulation obtained from a process for making an infant formula described above (or elsewhere in this specification).

Further benefits of the teachings of this specification will be apparent to one skilled in the art from reading this specification.

### DETAILED DESCRIPTION

This detailed description is intended only to acquaint others skilled in the art with Applicant's invention, its principles, and its practical application so that others skilled in the art may adapt and apply the invention in its numerous forms, as they may be best suited to the requirements of a particular use. This detailed description and its specific examples, while indicating certain embodiments, are intended for purposes of illustration only. This specification, therefore, is not limited to the described embodiments, and may be variously modified.

This specification relates to a process for crystallizing 2'-FL from a 2'-FL-containing solution. In general, this process comprises bringing a 2'-FL-containing solution to a supersaturated state with respect to 2'-FL, and then crystallizing the 2'-FL from the solution by evaporation until a crystallization mass is obtained which has a crystal yield of at least about 1% with respect to 2'-FL.

In some embodiments, the solution from which the 2'-FL is crystallized comprises a natural source of 2'-FL, such as, for example, an animal milk (*e.g*., human milk) or a composition derived from animal milk. In some embodiments, the solution from which the 2'-FL is crystallized is produced by a chemical synthesis. In still other embodiments, the 2'-FL-containing solution is obtained from a microbial fermentation process. For example, the 2'-FL source may be a fermentation broth obtained by microbial fermentation using a recombinant microorganism, such as bacteria or yeast. In some such embodiments, the microorganism is a yeast. In other embodiments, the microorganism is a bacteria. In some embodiments, the microorganism is *Escherichia coli.* In some embodiments, the fermentation occurs in a chemically defined medium.

Before crystallization, the source of 2'-FL (*e.g.,* fermentation broth) may be subjected to one or more purification processes. In some embodiments, for example, the 2'-FL source (*e.g.,* fermentation broth) is subjected to a centrifugation, sedimentation or one or more other process to remove cell biomass. In some embodiments, the 2'-FL source (*e.g.,* fermentation broth) is subjected to ultrafiltration. This can be helpful, for example, to remove cells and large biomolecules, such as proteins, nucleic acids and lipopolysaccharides. In some embodiments, the 2'-FL source (*e.g.,* fermentation broth) is subjected to nanofiltration. This can be helpful, for example, to concentrate the 2'-FL by reducing water while also removing minerals and various small biomolecules. In some embodiments, the 2'-FL source (*e.g.,* fermentation broth) is subjected to a chromatographic separation, such as gel-filtration chromatography. In some embodiments, the 2'-FL source (*e.g.,* fermentation broth) is subjected to microfiltration. This can be helpful, for example, to remove microbiological contamination. In some embodiments, the 2'-FL source (*e.g.,* fermentation broth) is subjected to cation exchange, anion exchange, ion exchange resin, mixed bed ion exchange and/or electrodialysis. These can be helpful, for example, to remove small charged molecules, salts and trace metals. In some embodiments, the 2'-FL source (*e.g.,* fermentation broth) is subjected to a de-coloration, by, for example, contacting it with powdered activated carbon or charcoal filtration. In some embodiments, the 2'-FL source (*e.g.,* fermentation broth) is subjected to evaporation. This can be helpful, for example, to concentrate the 2'-FL by removing water. In some embodiments, the 2'-FL source (*e.g.,* fermentation broth) is subjected to a combination of two or more of the above purification steps before the crystallization. Such embodiments may include use of the steps in various orders, as well as repeating various steps at different points in the process.

In some embodiments, for example, the 2'-FL source is a fermentation broth, which is subjected to ultrafiltration, cation exchange, anion exchange, mixed bed ion exchange and powdered activated carbon before the solution is brought to a supersaturated state for the crystallization. In some embodiments, the 2'-FL source is a fermentation broth, which is subjected to centrifugation, ultrafiltration, cation exchange, anion exchange, mixed bed ion exchange and powdered activated carbon before the solution is brought to a supersaturated state for the crystallization. In some embodiments, the 2'-FL source is a fermentation broth, which is subjected to ultrafiltration, nanofiltration, cation exchange, anion exchange, mixed bed ion exchange and powdered activated carbon before the solution is brought to a supersaturated state for the crystallization. In some embodiments, the 2'-FL source is a fermentation broth, which is subjected to centrifugation, ultrafiltration, nanofiltration, cation exchange, anion exchange, mixed bed ion exchange and powdered activated carbon before the solution is brought to a supersaturated state for the crystallization. In some embodiments, the 2'-FL source is a fermentation broth, which is subjected to ultrafiltration, nanofiltration, optional microfiltration, optional ion removal (*e.g.,* ion exchange resin and/or electrodialysis), optional pre-concentration (*e.g.,* evaporation or nanofiltration), decoloration (*e.g.,* charcoal filtration), microfiltration, and optional nanofiltration before the solution is brought to a supersaturated state for the crystallization.

In some embodiments, the 2'-FL concentration (or "purity") in the dry solids content of the syrup resulting from the pre-crystallization purification steps is less than 99%. In some embodiments, the 2'-FL concentration in the dry solids content of the syrup resulting from the pre-crystallization purification steps is less than 98%. In some embodiments, the 2'-FL concentration in the dry solids content of the syrup resulting from the pre-crystallization purification steps is less than about 95%. In some embodiments, the 2'-FL concentration in the dry solids content of the syrup resulting from the pre-crystallization purification steps is less than about 91%. In some embodiments, the 2'-FL concentration in the dry solids content of the syrup resulting from the pre-crystallization purification steps is less than about 90%. In some embodiments, the 2'-FL concentration in the dry solids content of the syrup resulting from the pre-crystallization purification steps is less than about 85%. In some embodiments, the 2'-FL concentration in the dry solids content of the syrup resulting from the pre-crystallization purification steps is less than about 80%. In some embodiments, the 2'-FL concentration in the dry solids content of the syrup resulting from the pre-crystallization purification steps is less than about 75%.

In some embodiments, the 2'-FL concentration in the dry solids content of the syrup resulting from the pre-crystallization purification steps is at least about 50%. In some embodiments, the 2'-FL concentration in the dry solids content of the syrup resulting from the pre-crystallization purification steps is at least about 55%. In some embodiments, the 2'-FL concentration in the dry solids content of the syrup resulting from the pre-crystallization purification steps is at least about 60%. In some embodiments, the 2'-FL concentration in the dry solids content of the syrup resulting from the pre-crystallization purification steps is at least about 65%. In some embodiments, the 2'-FL concentration in the dry solids content of the syrup resulting from the pre-crystallization purification steps is at least about 70%. In some embodiments, the 2'-FL concentration in the dry solids content of the syrup resulting from the pre-crystallization purification steps from the pre-crystallization purification steps is at least about 75%. In some embodiments, the 2'-FL concentration in the dry solids content of the syrup resulting from the pre-crystallization purification steps is at least about 80%. In some embodiments, the 2'-FL concentration in the dry solids content of the syrup resulting from the pre-crystallization purification steps is at least about 85%. In some embodiments, the 2'-FL concentration in the dry solids content of the syrup resulting from the pre-crystallization purification steps is at least about 90%. In some embodiments, the 2'-FL concentration in the dry solids content of the syrup resulting from the pre-crystallization purification steps is at least about 95%.

In some embodiments, the 2'-FL concentration in the dry solids content of the syrup resulting from the pre-crystallization purification steps is from about 60 to about 95%. In some embodiments, the 2'-FL concentration in the dry solids content of the syrup resulting from the pre-crystallization purification steps is from about 60 to about 90%. In some embodiments, the 2'-FL concentration in the dry solids content of the syrup resulting from the pre-crystallization purification steps is from about 65 to about 95%. In some embodiments, the 2'-FL concentration in the dry solids content of the syrup resulting from the pre-crystallization purification steps is from about 65 to about 90%. In some embodiments, the 2'-FL concentration in the dry solids content of the syrup resulting from the pre-crystallization purification steps is from about 70 to about 95%. In some embodiments, the 2'-FL concentration in the dry solids content of the syrup resulting from the pre-crystallization purification steps is from about 70 to about 90%. In some embodiments, the 2'-FL concentration in the dry solids content of the syrup resulting from the pre-crystallization purification steps is from about 70 to about 85%. In some embodiments, the 2'-FL concentration in the dry solids content of the syrup resulting from the pre-crystallization purification steps is from about 70 to about 80%. In some embodiments, the 2'-FL concentration in the dry solids content of the syrup resulting from the pre-crystallization purification steps is from about 70 to about 75%. In some embodiments, the 2'-FL concentration in the dry solids content of the syrup resulting from the pre-crystallization purification steps is from about 75 to about 95%. In some embodiments, the 2'-FL concentration in the dry solids content of the syrup resulting from the pre-crystallization purification steps is from about 80 to about 95%. In some embodiments, the 2'-FL concentration in the dry solids content of the syrup resulting from the pre-crystallization purification steps is from about 85 to about 95%. In some embodiments, the 2'-FL concentration in the dry solids content of the syrup resulting from the pre-crystallization purification steps is from about 90 to about 95%.

The above 2'-FL concentration percentages correspond to normalized peak area concentrations (or "purities") obtained using high performance liquid chromatography ("HPLC").

The 2'-FL is crystallized from a solution comprising 2'-FL and at least one other carbohydrate. For example, when the 2'-FL solution is derived from an enzymatic method or fermentative approach, the solution may contain, for example, a complex mixture of lactose, oligosaccharides in addition to the 2'-FL (*e.g.,* difucosyllactose ("DiFL")), monosaccharides, amino acids, polypeptides, proteins, monovalent and divalent salts, organic acids, nucleic acids, etc. In some embodiments, the total concentration of carbohydrates other than 2'-FL in the solution is at least about 1%. In some embodiments, the total concentration of carbohydrates other than 2'-FL in the solution is at least about 2%. In some embodiments, for example, the total concentration of carbohydrates other than 2'-FL in the solution is at least about 5%. In some embodiments, the concentration of DiFL in the solution is at least about 1%. In some embodiments, the concentration of DiFL in the solution is at least about 2%. In some embodiments, the concentration of DiFL in the solution is at least about 5%. In some embodiments, the concentration of DiFL in the solution is at least about 7%. In some embodiments, the concentration of DiFL in the solution is from about 1 to about 20%. In some embodiments, the concentration of DiFL in the solution is from about 2 to about 20%. In some embodiments, the concentration of DiFL in the solution is from about 2 to about 18%. In some embodiments, the concentration of DiFL in the solution is from about 5 to about 18%. In some embodiments, the concentration of DiFL in the solution is from about 2 to about 10%. In some embodiments, the concentration of DiFL in the solution is from about 5 to about 10%. In some embodiments, the concentration of DiFL in the solution is from about 10 to about 18%. In some embodiments, the concentration of lactose in the solution is no greater than about 0.1%. In some embodiments, the concentration of lactose in the solution is at least about 0.1%. In some embodiments, the concentration of lactose in the solution is at least about 2%. In some embodiments, the concentration of lactose in the solution is at least about 5%. In some embodiments, the concentration of lactose in the solution is from about 0.1 to about 15%. In some embodiments, the concentration of lactose in the solution is from about 0.1 to about 0.5%. In some embodiments, the concentration of lactose in the solution is from about 1 to about 12%. In some embodiments, the concentration of lactose in the solution is from about 2 to about 12%. In some embodiments, the concentration of lactose in the solution is from about 5 to about 12%. In some embodiments, the concentration of lactose in the solution is from about 2 to about 15%. In some embodiments, the concentration of lactose in the solution is from about 5 to about 15%. These percentages correspond to normalized peak area concentrations (or "purities") obtained using HPLC.

In general, the solution is brought to a supersaturated state before the crystallization. In some embodiments, the solution is brought to a 2'-FL supersaturation of greater than about 1.0. In some embodiments, the solution is brought to a 2'-FL supersaturation of from about 1.05 to about 1.8. In some embodiments, the solution is brought to a 2'-FL supersaturation of from about 1.1 to about 1.7. In some embodiments, the solution is brought to a 2'-FL supersaturation of from about 1.1 to about 1.5. In some embodiments, the solution is brought to a 2'-FL supersaturation of from about 1.2 to about 1.7. In some embodiments, the solution is brought to a 2'-FL supersaturation of from about 1.3 to about 1.7. In some embodiments, the solution is brought to a 2'-FL supersaturation of from about 1.3 to about 1.5. In this specification, unless stated otherwise, the terms "supersaturation" and "supersaturated" correspond to a dimensionless ratio of the measured 2'-FL content to the solubility of 2'-FL, the ratio being calculated from the following equation: $s = \frac{2 ′ - FL content in solution}{solubility of 2 ′ - FL at the same temperature}$ Here, s is supersaturation, and the unit of measurement for the 2'-FL content and 2'-FL solubility is g / 100 g of water. The solubility of 2'-FL at different temperatures is provided in the **Example 15**.

In some embodiments, the solution is brought to a supersaturated state by increasing the dry solids content to at least about 50% (by weight). In some such embodiments, the dry solids content is increased to at least about 55% (by weight). In some such embodiments, the dry solids content is increased to at least about 60% (by weight). In some such embodiments, the dry solids content is increased to at least about 65% (by weight). In some such embodiments, the dry solids content is increased to no greater than about 80% (by weight). In some such embodiments, the dry solids content is increased to no greater than about 78% (by weight). In some such embodiments, the dry solids content is increased to no greater than about 75% (by weight). In some such embodiments, the dry solids content is increased to from about 50 to about 80% (by weight). In some embodiments, the dry solids content is increased to from about 55 to about 78% (by weight). In some embodiments, the dry solids content is increased to from about 60 to about 75% (by weight). In some embodiments, the dry solids content is increased to from about 65 to about 75% (by weight). In some such embodiments, the dry solids content is increased to about 60% (by weight).

In some embodiments, the solution is brought to a supersaturated state with respect to 2'-FL by evaporation. In some such embodiments, the evaporation (or at least a portion thereof) is carried out at atmospheric pressure. In some embodiments, the evaporation (or at least a portion thereof) is carried out at a lower pressure. In some embodiments, the evaporation (or at least a portion thereof) is carried out at a pressure of no greater than about 250 mbar. In some embodiments, the evaporation (or at least a portion thereof) is carried out at a pressure of from about 120 to about 250 mbar. In some embodiments, the evaporation (or at least a portion thereof) is carried out at a pressure of from about 130 to about 220 mbar. In some embodiments, the evaporation (or at least a portion thereof) is carried out at a pressure of from about 150 to about 210 mbar

In general, the temperature at which the evaporation is carried out will depend on, for example, the pressure. In some embodiments, the temperature of the evaporation is the boiling temperature of the solution at the pressure. In some embodiments, the evaporation temperature is at least about 40°C. In some embodiments, the evaporation temperature is at least about 45°C. In some embodiments, the evaporation temperature is at least about 50°C. In some embodiments, the evaporation temperature is at least about 55°C. In some embodiments, the evaporation temperature is at least about 60°C. In some embodiments, the evaporation temperature is greater than 60°C. In some embodiments, the evaporation temperature is no greater than about 100°C. In some embodiments, the evaporation temperature is no greater than about 90°C. In some embodiments, the evaporation temperature is no greater than about 80°C. In some embodiments, the evaporation temperature is no greater than about 75°C. In some embodiments, the evaporation temperature is no greater than about 70°C (while temperatures greater than about 70°C can be advantageous for the evaporation, use of such temperatures can cause undesirable product degradation and/or color formation). In some embodiments, the evaporation temperature is from about 45 to about 80°C. In some embodiments, the evaporation temperature is from about 50 to about 80°C. In some embodiments, the evaporation temperature is from about 55 to about 80°C. In some embodiments, the evaporation temperature is from about 60 to about 80°C. In some embodiments, the evaporation temperature is greater than 60 to no greater than about 80°C. In some embodiments, the evaporation temperature is from about 60 to about 75°C. In some embodiments, the evaporation temperature is greater than 60 to no greater than about 75°C. In some embodiments, the evaporation temperature is from about 65 to about 75°C. In some embodiments, the evaporation temperature is from about 40 to about 70°C. In some embodiments, the evaporation temperature is from about 45 to about 70°C. In some embodiments, the evaporation temperature is from about 50 to about 70°C. In some embodiments, the evaporation temperature is from about 55 to about 70°C. In some embodiments, the evaporation temperature is from about 60 to about 70°C. In some embodiments, the evaporation temperature is greater than 60 to no greater than about 70°C.

In some embodiments, seeding is used to initiate formation of 2'-FL crystals from the supersaturated solution. In some such embodiments, seeding is effected by adding seed crystals to the supersaturated solution. In some such embodiments, the seed crystals comprise a particulate 2'-FL powder. In some embodiments, the seed crystals comprise anhydrous 2'-FL seed crystals.

Seed crystals can be made by various processes, including, for example, those discussed in this specification. In some embodiments, the dry seeds are milled to get smaller particle size. The seed crystals can be used also in suspension form.

The desired amount of seed crystals may depend on, for example, the size of the seed crystals.

In some embodiments, the amount of dry seed crystals used is from about 0.001 to about 1% (by weight) of particulate 2'-FL, based on the 2'-FL of the crystallization mass.

In some embodiments, a suspension of seed crystals is used, and the amount of suspension seed crystals is from about 1 to about 30% (by weight) of particulate 2'-FL, based on the 2'-FL of the crystallization mass.

In some embodiments, crystallization is initiated without adding 2'-FL seed crystals to the supersaturated solution. In some such embodiments, for example, seeding is effected using spontaneous seeding or ultrawave seeding.

In general, initiation of crystallization (*e.g.,* addition of seed crystals) is carried out when a suitable supersaturation has been achieved. In some embodiments, initiation of crystallization (*e.g.,* addition of seed crystals) is carried out when the 2'-FL supersaturation is greater than about 1.0. In some embodiments, initiation of crystallization (*e.g.,* addition of seed crystals) is carried out when the 2'-FL supersaturation is from about 1.05 to about 1.8. In some embodiments, initiation of crystallization (*e.g.,* addition of seed crystals) is carried out when the 2'-FL supersaturation is from about 1.1 to about 1.7. In some embodiments, initiation of crystallization (*e.g.,* addition of seed crystals) is carried out when the 2'-FL supersaturation is from about 1.1 to about 1.5. In some embodiments, initiation of crystallization (*e.g.,* addition of seed crystals) is carried out when the 2'-FL supersaturation is from about 1.2 to about 1.7. In some embodiments, initiation of crystallization (*e.g.,* addition of seed crystals) is carried out when the 2'-FL supersaturation is from 1.3 to about 1.7. In some embodiments, initiation of crystallization (*e.g.,* addition of seed crystals) is carried out when the 2'-FL supersaturation is from about 1.3 to about 1.5.

In some embodiments, initiation of crystallization (*e.g.,* addition of seed crystals) is carried out when the dry solids content of the syrup at least about 60% (by weight). In some embodiments, initiation of crystallization (*e.g.,* addition of seed crystals) is carried out when the dry solids content of the syrup at least about 65% (by weight). In some embodiments, initiation of crystallization (*e.g.,* addition of seed crystals) is carried out when the dry solids content of the syrup is no greater than about 80% (by weight). In some embodiments, initiation of crystallization (*e.g.,* addition of seed crystals) is carried out when the dry solids content of the syrup is no greater than about 75% (by weight). In some embodiments, initiation of crystallization (*e.g.,* addition of seed crystals) is carried out when the dry solids content of the syrup is from about 60 to about 80% (by weight). In some embodiments, initiation of crystallization (*e.g.,* addition of seed crystals) is carried out when the dry solids content of the syrup is from about 65 to about 80% (by weight). In some embodiments, initiation of crystallization (*e.g.,* addition of seed crystals) is carried out when the dry solids content of the syrup is from about 65 to about 77% (by weight). In some embodiments, initiation of crystallization (*e.g.,* addition of seed crystals) is carried out when the dry solids content of the syrup is from about 65 to about 75% (by weight). In some embodiments, initiation of crystallization (*e.g.,* addition of seed crystals) is carried out when the dry solids content of the syrup is from about 65 to about 70% (by weight). In some embodiments, initiation of crystallization (*e.g.,* addition of seed crystals) is carried out when the dry solids content of the syrup is from about 70 to about 76% (by weight).

Once crystallization is initiated, the crystallization may generally be carried out at the boiling point of the solution.

In some embodiments, at least a portion (or all) of the crystallization is carried out at atmospheric pressure.

In some embodiments, at least a portion of the crystallization is carried out at a pressure that is less than atmospheric pressure. In some such embodiments, the crystallization (or at least a portion thereof) is carried out at a pressure of no greater than about 250 mbar. In some embodiments, the crystallization (or at least a portion thereof) is carried out at a pressure of from about 50 to about 250 mbar. In some embodiments, the crystallization (or at least a portion thereof) is carried out at a pressure of from about 50 to about 120 mbar. In some embodiments, the crystallization (or at least a portion thereof) is carried out at a pressure of from about 120 to about 250 mbar. In some embodiments, the crystallization (or at least a portion thereof) is carried out at a pressure of from about 130 to about 220 mbar. In some embodiments, the crystallization (or at least a portion thereof) is carried out at a pressure of from about 150 to about 210 mbar. In some embodiments, the crystallization (or at least a portion thereof) is carried out at a pressure of from about 170 to about 205 mbar.

In some embodiments, at least about 50% of the crystallization is carried out at a sub-atmospheric pressure range discussed above. In some embodiments, at least about 75% of the crystallization is carried out at a sub-atmospheric pressure range discussed above. In some embodiments, at least about 90% of the crystallization is carried out at a sub-atmospheric pressure range discussed above. In some embodiments, at least about 95% of the crystallization is carried out at a sub-atmospheric pressure range discussed above. In some embodiments, at least about 98% of the crystallization is carried out at a sub-atmospheric pressure range discussed above. In some embodiments, at least about 99% of the crystallization is carried out at a sub-atmospheric pressure range discussed above. In some embodiments, the entire crystallization is carried out at a sub-atmospheric pressure range discussed above.

In some embodiments, at least a portion of the crystallization is carried out at the boiling temperature of the solution at the pressure.

At least a portion of the crystallization is carried out at a temperature of greater than 60°C. In some embodiments, at least a portion of the crystallization is carried out at a temperature of no greater than about 100°C. In some embodiments, at least a portion of the crystallization is carried out at a temperature of no greater than about 90°C. In some embodiments, at least a portion of the crystallization is carried out at a temperature of no greater than about 80°C. In some embodiments, at least a portion of the crystallization is carried out at a temperature of no greater than about 75°C. In some embodiments, at least a portion of the crystallization is carried out at a temperature of no greater than about 70°C (while temperatures greater than about 70°C can be advantageous for crystallization, use of such temperatures can cause undesirable product degradation and/or color formation

At least a portion of the crystallization is carried out at a temperature of from about 60 to about 80°C. In some embodiments at least a portion of the crystallization is carried out at a temperature of greater than 60 and no greater than about 80°C. In some embodiments, at least a portion of the crystallization is carried out at a temperature of from about 60 to about 75°C. In some embodiments at least a portion of the crystallization is carried out at a temperature of greater than 60 and no greater than about 75°C. In some embodiments, at least a portion of the crystallization is carried out at a temperature of from about 65 to about 75°C.

In some embodiments, at least a portion of the crystallization is carried out at a temperature of from about 60 to about 70°C. In some embodiments at least a portion of the crystallization is carried out at a temperature of greater than 60 and no greater than about 70°C.

In some embodiments, at least about 50% of the crystallization is carried out at a temperature range discussed above. In some embodiments, at least about 75% of the crystallization is carried out at a temperature range discussed above. In some embodiments, at least about 90% of the crystallization is carried out at a temperature range discussed above. In some embodiments, at least about 95% of the crystallization is carried out at a temperature range discussed above. In some embodiments, at least about 98% of the crystallization is carried out at a temperature range discussed above. In some embodiments, at least about 99% of the crystallization is carried out at a temperature range discussed above. In some embodiments, the entire crystallization is carried out at a temperature range discussed above.

In some embodiments, the pH of the crystallization is from about 4 to about 11. In some embodiments, the pH during at least a portion of the crystallization is from about 4 to about 8. In some embodiments, the pH during at least a portion of the crystallization is from about 5 to about 7. In some embodiments, the pH during at least a portion of the crystallization is from about 5 to about 6. In some embodiments, the pH during all the crystallization is no greater than about 7. In some embodiments, the pH during all the crystallization is from about 5 to about 7. In some embodiments, the pH during all the crystallization is from about 5 to about 6.

During the crystallization, the crystal mass is typically mechanically mixed. In some embodiments, the mixing is optimally arranged to maximize homogeneity throughout the entire volume of the crystallization mass. This generally provides for desirable heat and mass transfer and small supersaturation gradients throughout whole crystallization volume. In some embodiments, this is achieved using traditional rotating mixer blades. In general, the intensity of the mixing is moderated to prevent excess of fines formation.

During the crystallization, the crystal suspension is generally subjected to boiling and evaporation until a sufficient degree of crystallization (yield, reduction in 2'-FL purity of the mother liquor and/or crystal size) has been achieved. In some embodiments, the crystallization by evaporation is continued for from about 1 to about 50 hr. In some embodiments, the crystallization by evaporation is continued for from about 2 to about 40 hr. In some embodiments, the crystallization by evaporation is continued for from about 2 to about 30 hr. In some embodiments, the crystallization by evaporation is continued for from about 2 to about 20 hr. In some embodiments, the crystallization by evaporation is continued for from about 2 to about 15 hr. In some embodiments, the crystallization by evaporation is continued for from about 2 to about 5 hr.

It is typically desirable to achieve a 2'-FL crystal yield of from about 1 to about 80% (this yield being the crystal yield of 2'-FL immediately after boiling). In some embodiments, the target yield is the maximum crystallization yield that allows for effective crystal separation, washing and drying. More specifically, low crystal yields are typically undesirable because they create losses in manufacturing capacity and efficiency. However, increases in crystal yield cause the viscosity of the product suspension to increase. A high viscosity, in turn, can create difficulty (and, in some instances, even prevent) crystal separation. Thus, in some embodiments, the target yield is the maximum yield corresponding to a viscosity allowing for cost-effective separation.

In some embodiments, a crystallization mass is obtained which has a crystal yield of from about 1 to about 80% with respect to 2'-FL. In some embodiments, a crystallization mass is obtained which has a crystal yield of from about 30 to about 80% with respect to 2'-FL. In some embodiments, a crystallization mass is obtained which has a crystal yield of from about 50 to about 80% with respect to 2'-FL. In some embodiments, a crystallization mass is obtained which has a crystal yield of from about 55 to about 75% with respect to 2'-FL. In some embodiments, a crystallization mass is obtained which has a crystal yield of from about 60 to about 75% with respect to 2'-FL. The crystal yields above refer to yields immediately after boiling.

In some embodiments, the crystal size (*i.e.,* the largest dimension of a crystal) of the main crystal population is at least about 1 µm. In some embodiments, the crystal size of the main crystal population is at least about 5 µm. In some embodiments, the crystal size of the main crystal population is at least about 10 µm. In some embodiments, the crystal size of the main crystal population is at least about 20 µm. In some embodiments, the crystal size of the main crystal population is at least about 30 µm. In some embodiments, the crystal size of the main crystal population is no greater than about 10000 µm. In some embodiments, the crystal size of the main crystal population is no greater than about 5000 µm. In some embodiments, the crystal size of the main crystal population is no greater than about 1000 µm. In some embodiments, the crystal size of the main crystal population is no greater than about 500 µm. In some embodiments, the crystal size of the main crystal population is no greater than about 300 µm. In some embodiments, the crystal size of the main crystal population is no greater than about 200 µm. In some embodiments, the crystal size of the main crystal population ranges from about 1 to about 10000 µm. In some embodiments, the crystal size of the main crystal population ranges from about 10 to about 500 µm. In some embodiments, the crystal size of the main crystal population ranges from about 30 to about 200 µm. In some embodiments, the crystal size of the main crystal population ranges from about 30 to about 100 µm. In some embodiments, the crystal size of the main crystal population ranges from about 100 to about 200 µm. As used in this specification, the term "main crystal population" is a visual judgement of a microscopic image from a crystallization mass. Outlier crystals that are either abnormally small or abnormally large are ignored.

In some embodiments, additional feed liquid is added to the 2'-FL solution simultaneously with the crystallization by evaporation to increase the level of the 2'-FL solution in the crystallizer and/or to increase the dry substance content of the 2'-FL solution. In some embodiments, the additional feed liquid is added continuously. In some embodiments, the additional feed liquid is added batchwise.

In some embodiments, the 2'-FL-containing solution is cooled simultaneously with the crystallization. In some embodiments, the crystallization comprises a combined boiling- and-cooling process. In such a process, the temperature may, for example, be decreased to a temperature of from about 10 to about 20°C less than the seeding point temperature. In some such embodiments, the cooling rate is from about 1°C/hr to about 5°C/hr.

In some embodiments, when the crystallization by evaporation is terminated, the crystal mass is mixed before allowing the mass to cool. In some such embodiments, the mixing is carried out under, for example, atmospheric pressure, at a temperature of from about 40 to about 75°C. In some such embodiments, the mixing is carried out under, for example, atmospheric pressure, at a temperature of from about 40 to about 70°C. In some embodiments, the mixing is carried out under, for example, atmospheric pressure, at a temperature of from about 45 to about 70°C. In some embodiments, the mixing is carried out under, for example, atmospheric pressure, at a temperature of from about 50 to about 70°C. In some embodiments, the mixing is carried out under, for example, atmospheric pressure, at a temperature of from about 55 to about 70°C. In some embodiments, the mixing is carried out under, for example, atmospheric pressure, at a temperature of from about 60 to about 70°C. In some embodiments, the mixing is carried out under, for example, atmospheric pressure, at a temperature of greater than 60 and no greater than about 70°C. In some embodiments, the mixing is continued for from about 0.5 to about 30 hr.

In some embodiments, when the crystallization by evaporation is terminated, the temperature of the crystallization mass is decreased to room temperature. In some embodiments, when the crystallization by evaporation is terminated, the temperature of the crystallization mass is decreased to about 20°C. In some embodiments, a longer cooling period is used when the 2'-FL content is low.

In some embodiments, the cooling is conducted over a period of up to about 3 days. In some embodiments, the cooling is conducted over a period of from about 1 to about 3 days. In some embodiments, the cooling is conducted over a period of from about 1 to about 30 hr.

In some embodiments, the cooling rate is from about 1°C/h to about 5°C/h.

In some embodiments, the apparent viscosity of the crystallization mass during the crystallization by evaporation is from about 3 to about 200 Pa·s. In some embodiments, the apparent viscosity of the crystallization mass during the crystallization by evaporation is from about 5 to about 200 Pa·s. In some embodiments, the apparent viscosity of the crystallization mass during the crystallization by evaporation is from about 5 to about 100 Pa·s. In some embodiments, the apparent viscosity of the crystallization mass during the crystallization by evaporation is from about 50 to about 100 Pa·s. In some embodiments, the apparent viscosity of the crystallization mass during the crystallization by evaporation is from about 5 to about 50 Pa·s. In some embodiments, the apparent viscosity of the crystallization mass during the crystallization by evaporation is from about 5 to about 30 Pa·s. A viscosity in these ranges is generally suitable for effective separation of the crystallized material. If the crystallization mass becomes too viscous for effective separation, the viscosity of the crystallization mass may be decreased by, for example, decreasing the supersaturation of the crystallization mass. The supersaturation of the crystallization mass may be decreased by, for example, raising the temperature and/or diluting the crystallization mass with water or a 2'-FL-containing solution.

The crystals can be separated by, for example, centrifugation, filtration, decantation etc. In some embodiments, separation of the crystals comprises centrifugation.

Following separation, the crystals are typically dried by, for example, contacting them with hot air. In some embodiments, the crystals are washed following separation. In some embodiments, the crystals are washed with water and then dried by, for example, contacting them with hot air. In some embodiments, the water content of the resulting crystal fraction is less than about 1% (by weight). In some embodiments, the water content of the resulting crystal fraction is less than about 0.5% (by weight). Water content may be measured by weight loss on drying or by Karl Fisher titration.

In some embodiments, solid-liquid-separation ("SLS") is performed on the crystalline product to remove micro-impurities, such as traces of protein and DNA, amino acids, carbohydrate impurities, trace elements, etc.

In some embodiments, the 2'-FL content of the resulting crystal fraction of this process is at least about 85%. In some embodiments, the 2'-FL content of the resulting crystal fraction is at least about 90%. In some embodiments, the 2'-FL content of the resulting crystal fraction is at least about 91%. In some embodiments, the 2'-FL content of the resulting crystal fraction is at least about 92%. In some embodiments, the 2'-FL content of the resulting crystal fraction is at least about 93%. In some embodiments, the 2'-FL content of the resulting crystal fraction is at least about 94%. In some embodiments, the 2'-FL content of the resulting crystal fraction is at least about 95%. In some embodiments, the 2'-FL content of the resulting crystal fraction is at least about 96%. In some embodiments, the 2'-FL content of the resulting crystal fraction is at least about 97%. In some embodiments, the 2'-FL content of the resulting crystal fraction is at least about 98%. In some embodiments, the 2'-FL content of the resulting crystal fraction is at least about 99%. In some embodiments, the 2'-FL content of the resulting crystal fraction is at least about 99.5%. In some embodiments, the 2'-FL content of the resulting crystal fraction is at least about 99.9%. These percentages correspond to normalized peak area concentrations or purities obtained using HPLC.

In some embodiments, the melting point of the resulting crystals of this process is from about from about 230 to about 239°C. In some embodiments, the melting point of the resulting crystals of this process is from about 230 to about 232°C. In some embodiments, the melting point of the resulting crystals of this process is from about 230 to about 231°C. In some embodiments, the melting point of the resulting crystals of this process is from about 232 to about 239°C. In some embodiments, the melting point of the resulting crystals of this process is from about 232 to about 238°C. In some embodiments, the melting point of the resulting crystals of this process is from about 233 to about 238°C. In some embodiments, the melting point of the resulting crystals of this process is from about 234 to about 237°C. In some embodiments, the melting point of the resulting crystals of this process is less than 236°C. In some embodiments, the melting point of the resulting crystals of this process is at least about 233°C and less than 236°C. In some embodiments, the melting point of the resulting crystals of this process is at least about 234°C and less than 236°C. In some embodiments, the melting point of the resulting crystals of this process is from about 233°C to 235°C. The above melting points correspond to a melting point determined with a 1°C/min heating rate using the European Pharmacopoeia capillary melting point method.

In some embodiments, the resulting crystals exhibit X-ray powder diffraction spectra discussed in International Patent Appl. Publ. No. WO2011/150939 for the crystalline form cited in WO2011/150939 as "crystalline 2'-O-fucosyllactose polymorph II."

In some embodiments, the resulting crystals exhibit an X-ray powder diffraction reflection, based on a measurement using CuKa radiation, at 16.98±0.20, 13.65±0.20 and 18.32±0.20 2Θ angles. In some embodiments, the resulting crystals exhibit an X-ray powder diffraction reflection, based on a measurement using CuKa radiation, at 16.98±0.20, 13.65±0.20, 18.32±0.20 and 21.70±0.20 2Θ angles. In some embodiments, the resulting crystals exhibit an X-ray powder diffraction reflection, based on a measurement using CuKa radiation, at 16.98±0.20, 13.65±0.20, 18.32±0.20, 21.70±0.20 and 15.22±0.20 2Θ angles. In some embodiments, the resulting crystals exhibit an X-ray powder diffraction reflection, based on a measurement using CuKa radiation, at 16.98±0.20, 13.65±0.20, 18.32±0.20, 21.70±0.20, 15.22±0.20 and 20.63±0.20 2Θ angles. In some embodiments, the resulting crystals exhibit an X-ray powder diffraction reflection, based on a measurement using CuKa radiation, at 16.98±0.20, 13.65±0.20, 18.32±0.20, 21.70±0.20, 15.22±0.20, 20.63±0.20 and 11.94±0.20 2Θ angles.

In some embodiments, recrystallization is performed one or more times to increase 2'-FL purity. Recrystallization may be carried out by, for example, dissolving the 2'-FL crystals in water (typically deionized water), bringing the resulting solution to a supersaturated state with respect to 2'-FL (via, for example, evaporation), and crystallizing using the crystallization-by-evaporation methodology described above.

In some embodiments, yield is increased by performing crystallization of the mother liquor produced by the initial crystallization. Such a crystallization may be carried out by, for example, bringing the mother liquor to a supersaturated state with respect to 2'-FL (via, for example, evaporation), and crystallizing using the crystallization-by-evaporation methodology described above.

In some embodiments, the 2'-FL crystalline product of the process described in this specification is incorporated into a food (*e.g.,* human or pet food), dietary supplement or medicine. In some embodiments, the crystalline product is incorporated into a human baby food (*e.g.,* infant formula). In some such embodiments, the 2'-FL is mixed with other ingredients of the food, dietary supplement or medicine. When incorporated into an infant formula, for example, the 2'-FL may be mixed with other infant formula ingredients such as, for example, nonfat milk, a carbohydrate source (*e.g.,* lactose), a protein source (*e.g.,* whey protein concentrate), a fat source (*e.g.,* high oleic safflower oil), vitamins, minerals, etc. In some embodiments, the 2'-FL concentration in the infant formula is approximately the same concentration as the 2'-FL concentration generally present in human breast milk.

In general, this process may be used to produce an anhydrous crystalline product. In some embodiments, the moisture content of the dried 2'-FL crystal product, as measured by Karl Fischer titration, is less than 1.0% (by weight). In some such embodiments, the moisture content of the dried 2'-FL crystal product is less than 0.9% (by weight). In some embodiments, the moisture content of the dried 2'-FL crystal product is less than 0.8% (by weight). In some embodiments, the moisture content of the dried 2'-FL crystal product is less than 0.7% (by weight). In some embodiments, the moisture content of the dried 2'-FL crystal product is less than 0.6% (by weight). In some embodiments, the moisture content of the dried 2'-FL crystal product is less than 0.5% (by weight). In some embodiments, the moisture content of the dried 2'-FL crystal product is less than 0.4% (by weight). In some embodiments, the moisture content of the dried 2'-FL crystal product is less than 0.3% (by weight). In some embodiments, the moisture content of the dried 2'-FL crystal product is less than 0.2% (by weight). In some embodiments, the moisture content of the dried 2'-FL crystal product is less than 0.1% (by weight). In some embodiments, no moisture is detected in the dried 2'-FL crystal product, as measured by Karl Fischer titration.

In some embodiments, the 2'-FL crystalline product of the process described in this specification is converted into a different physical form for an intended application. For example, in some embodiments, the crystalline product is dissolved or suspended in water or other solvent to form a solution or suspension, which, in turn, is incorporated into (or otherwise used in the manufacture of) a food (*e.g.,* infant formula), dietary supplement or medicine. In other embodiments, the crystalline product is dissolved in water or other solvent to form a solution, which is subsequently spray dried to form an amorphous 2'-FL composition, which, in turn, is incorporated into (or otherwise used in the manufacture of) a food (*e.g.,* infant formula), dietary supplement or medicine.

In some embodiments, when the 2'-FL product is incorporated into a food, dietary supplement or medicine, the amount of the 2'-FL is an amount that is effective to convey a benefit, such as a prebiotic, antibacterial, antiviral, immune system enhancing and/or brain development enhancing effect.

The crystallization described in this specification does not require an organic solvent to be present in the solution.

In some embodiments, no alcohol (*e.g.,* methanol, ethanol, etc.) is added to the solution from which the 2'-FL is crystallized. In some embodiments, no alcohol is added while the solution is being brought to supersaturation with respect to 2'-FL. In some embodiments, no alcohol is added while evaporation is being used to bring the solution to supersaturation with respect to 2'-FL. In some embodiments, no alcohol is added while 2'-FL crystallization is occurring. In some embodiments, the total concentration of any alcohol present in the solution while the solution is being brought to supersaturation with respect to 2'-FL is no greater than about 1% (by weight). In some embodiments, the total concentration of any alcohol present in the solution while evaporation is being used to bring the solution to supersaturation with respect to 2'-FL is no greater than 1% (by weight). In some embodiments, the total concentration of any alcohol present in the solution while 2'-FL crystallization is occurring is no greater than about 1% (by weight). In some embodiments, the total concentration of any alcohol present in the solution while the solution is being brought to supersaturation with respect to 2'-FL is no greater than about 0.1% (by weight). In some embodiments, the total concentration of any alcohol present in the solution while evaporation is being used to bring the solution to supersaturation with respect to 2'-FL is no greater than 0.1% (by weight). In some embodiments, the total concentration of any alcohol present in the solution while 2'-FL crystallization is occurring is no greater than about 0.1% (by weight). In some embodiments, the solution consists essentially of no alcohol while the solution is being brought to supersaturation with respect to 2'-FL. In some embodiments, the solution consists essentially of no alcohol while evaporation is being used to bring the solution to supersaturation with respect to 2'-FL. In some embodiments, the solution consists essentially of no alcohol while 2'-FL crystallization is occurring. Here, the phrase "consists essentially of no alcohol" means the amount of alcohol present in the solution (if any is present at all) is insufficient to materially affect the crystallization.

In some embodiments, no organic acid solvent (*e.g.,* acetic acid) is added to the solution from which the 2'-FL is crystallized. In some embodiments, no organic acid solvent is added while the solution is being brought to supersaturation with respect to 2'-FL. In some embodiments, no organic acid solvent is added while evaporation is being used to bring the solution to supersaturation with respect to 2'-FL. In some embodiments, no organic acid solvent is added while 2'-FL crystallization is occurring. In some embodiments, the total concentration of any organic acid solvent present in the solution while the solution is being brought to supersaturation with respect to 2'-FL is no greater than about 1% (by weight). In some embodiments, the total concentration of any organic acid solvent present in the solution while evaporation is being used to bring the solution to supersaturation with respect to 2'-FL is no greater than 1% (by weight). The total concentration of any organic acid solvent present in the solution while 2'-FL crystallization is occurring is no greater than about 1% (by weight). In some embodiments, the total concentration of any organic acid solvent present in the solution while the solution is being brought to supersaturation with respect to 2'-FL is no greater than about 0.1% (by weight). In some embodiments, the total concentration of any organic acid solvent present in the solution while evaporation is being used to bring the solution to supersaturation with respect to 2'-FL is no greater than 0.1% (by weight). In some embodiments, the total concentration of any organic acid solvent present in the solution while 2'-FL crystallization is occurring is no greater than about 0.1% (by weight). In some embodiments, the solution consists essentially of no organic acid solvent while the solution is being brought to supersaturation with respect to 2'-FL. In some embodiments, the solution consists essentially of no organic acid solvent while evaporation is being used to bring the solution to supersaturation with respect to 2'-FL. In some embodiments, the solution consists essentially of no organic acid solvent while 2'-FL crystallization is occurring. Here, the phrase "consists essentially of no organic acid solvent" means the amount of organic acid solvent present in the solution (if any is present at all) is insufficient to materially affect the crystallization.

In some embodiments, no organic solvent is added to the solution from which the 2'-FL is crystallized. In some embodiments, no organic solvent is added while the solution is being brought to supersaturation with respect to 2'-FL. In some embodiments, no organic solvent is added while evaporation is being used to bring the solution to supersaturation with respect to 2'-FL. In some embodiments, no organic solvent is added while 2'-FL crystallization is occurring. In some embodiments, the total concentration of any organic solvent present in the solution while the solution is being brought to supersaturation with respect to 2'-FL is no greater than about 1% (by weight). In some embodiments, the total concentration of any organic solvent present in the solution while evaporation is being used to bring the solution to supersaturation with respect to 2'-FL is no greater than 1% (by weight). The total concentration of any organic solvent present in the solution while 2'-FL crystallization is occurring is no greater than about 1% (by weight). In some embodiments, the total concentration of any organic solvent present in the solution while the solution is being brought to supersaturation with respect to 2'-FL is no greater than about 0.1% (by weight). In some embodiments, the total concentration of any organic solvent present in the solution while evaporation is being used to bring the solution to supersaturation with respect to 2'-FL is no greater than 0.1% (by weight). In some embodiments, the total concentration of any organic solvent present in the solution while 2'-FL crystallization is occurring is no greater than about 0.1% (by weight). In some embodiments, the solution consists essentially of no organic solvent while the solution is being brought to supersaturation with respect to 2'-FL. In some embodiments, the solution consists essentially of no organic solvent while evaporation is being used to bring the solution to supersaturation with respect to 2'-FL. In some embodiments, the solution consists essentially of no organic solvent while 2'-FL crystallization is occurring. Here, the phrase "consists essentially of no organic solvent" means the amount of organic solvent present in the solution (if any is present at all) is insufficient to materially affect the crystallization.

### EXAMPLES

The following examples are merely illustrative, and not limiting to the remainder of this specification in any way.

In the below examples, the following definitions are used:

A value identified by "[1]" refers to normalized peak area purity or concentration obtained using HPLC (CarboPac PA100, Thermo Fisher Scientific, Waltham, MA USA). This is a percentage of peak area relative to the total area of peaks.

A value identified by "[2]" refers to 2'-FL yield calculated from the purity values.

A value identified by "[3]" refers to dry solid content ("DS") as measured by Karl Fischer titration.

A value identified by "[4]" refers to dry solid content calculated from a mass balance based on the initial dry solid content measured by using Karl Fischer titration and the amount of water evaporated or added.

A value identified by "[5]" refers to moisture content measured by weight loss on drying.

A value identified by "[6]" refers to a crystal size measurement (*i.e.,* measurement of the largest dimension of a crystal) made from a microscopic image. Outlier crystals that are either abnormally small or abnormally large are ignored.

A value identified by "[7]" refers to a sugar color under the International Commission for Uniform Process of Sugar Analysis ("ICUMSA") sugar color grading system.

A value identified by "[8]" refers to a supersaturation with respect to 2'-FL defined as a ratio between 2'-FL concentration in solution (g / 100 g water) and solubility of 2'-FL in water (g / 100 g water) at the same temperature.

A value identified by "[9]" is the melting point measured with a 1°C/min heating rate using the European Pharmacopoeia capillary melting point method.

A value identified by "[10]" refers to normalized peak area purity or concentration obtained using HPLC (X-Bridge Amide HILIC column, Waters, Milford, MA USA). This is a percentage of peak area relative to the total area of peaks.

The abbreviation "HPLC" refers to high performance liquid chromatography.

The abbreviation "DiFL" refers to difucosyllactose.

### Example 1: Illustration of 2'-FL Seed Crystal Preparation

An aqueous feed solution, which had a 2'-FL concentration of 90.4% [1], a DiFL concentration of 8.9% [1], and a lactose concentration of 0.2% [1], was evaporated to a DS of 84.4% [3] (Rotavapor R-151 evaporator). Ethanol (279 g) was then added to the syrup (415 g) by mixing the solution by hand with an anchor agitator. The resulting solution was kept in a rotating Rotavapor bottle at 60°C for 2.3 hr. During this time, water (10 g) was added twice (20 g in total) to dilute the solution. The first water addition occurred 1.0 hr after the ethanol addition, and the second water addition occurred 1.6 hr after the ethanol addition.

A portion of the resulting syrup (306 g) was moved to a glass reactor. Ethanol (71 g) was added at 60°C. Crystals began to form. The crystallizing solution was stirred at 60°C for 1.2 hr, cooled to 55°C within 1 hr, and further to 15°C within 15 hr under continuous stirring. After cooling, the obtained crystal mass continued to be stirred at 15°C for 3.9 hr.

The crystal mass was centrifuged with a batch-wise centrifuge having 22.5 cm basket diameter. For this centrifugation, 343 g of crystal mass was loaded onto the centrifuge at the acceleration stage at a 500 rpm rotation speed. Wash water (75 mL) was added at a 2000 rpm rotation speed, and then centrifugation was continued for 5 min at a rotation speed of 3500 rpm. The resulting centrifuged crystals (85 g) were dried in a heating chamber at 50°C for 3.4 hr. The moisture content after drying was 0.8% [3].

Crystals prepared in accordance with the process illustrated here were used in **Example 2**. Before being used, the crystals were washed with ethanol, dried and then grinded in a porcelain mill to break the crystal structure and provide irregular shaped particles to initiate secondary nucleation. The washed crystals had a 2'-FL purity of 94.0% [1].

### Example 2: Preparation of Seed Crystals Using Seed Crystals Prepared in Accordance with Example 1

An aqueous feed solution, which had a 2'-FL concentration of 90.7% [1], DiFL concentration of 9.3% [1], and lactose concentration of <0.1% [1], was evaporated to a DS of 82.9% [3] (Rotavapor R-151 evaporator). The resulting syrup (683 g) was moved to a glass reactor, and seeded with 0.29 g of 2'-FL dry seed crystals (prepared in accordance with **Example 1**) at a temperature of 55°C.

The seeded syrup was stirred while being maintained at 55°C. Ethanol was added in two portions: the first portion (610 g) was added 0.9 hr after seeding within 0.3 hr, and the second portion (221 g) was added 2.3 hr after seeding. The crystallizing solution was then cooled from 55°C to 15°C within 17 hr while stirring. After cooling to 15°C, the stirring was continued at 15°C for 0.9 hr. The resulting crystal mass (1416 g) was centrifuged with 100 mL wash water (batchwise centrifuge, basket diameter 22.5 cm, 3500 rpm, 5 min). A total of 557 g of crystal cake was obtained. Of this, 241 g were dried in a heating chamber at 60°C for 2.3 hr. The moisture content after drying was 0.8 % [3].

Crystals prepared in accordance with this process were, in turn, used as seed crystals in **Example 3** below. Before being used, the crystals were washed with ethanol, dried and then grinded in a porcelain mill. The washed crystals had a 2'-FL purity of 91.6% [1].

### Example 3: Illustration of 2'-FL Crystallization

An aqueous feed solution, which had a 2'-FL concentration of 89.3% [1], a DiFL concentration of 9.5% [1], and a lactose concentration of <0.1% [1], was evaporated to a DS of 61.0% [4] (Rotavapor R-151 evaporator). The syrup (1540 g) was then seeded with 0.5 g of 2'-FL dry seed crystals (preparation described in **Example 2**) at a temperature of 57°C (supersaturation of 1.01 [8]). This resulted in very little crystal formation. Consequently, the seeded syrup was kept boiling at a temperature of 64°C and pressure of 130-180 mbar for 2.8 hr. The syrup was then seeded a second time with 0.5 g of 2'-FL dry seed crystals at a DS of 67.2% [4] at a temperature of 65°C (supersaturation of 1.27 [8]). After the second seeding, crystallization occurred. The boiling crystallization was continued at a temperature of 65°C and pressure of 175 mbar for 3.3 hr.

The obtained crystal mass, which had a DS of 72.8% [3], was allowed to cool to room temperature (21.5°C) and then kept in a rotating Rotavapor bottle overnight (18 hr). After 21.5 hr from the first seeding, a small sample of the mass was centrifuged without washing (Hettich Rotanta 460R centrifuge, 2500 rpm, 10 min) to determine the crystal content. The crystal cake 2'-FL purity was 96.7% [1] and the mother liquor 2'-FL purity was 85.6% [1]. This corresponds to 36% 2'-FL yield [2]. The viscosity of the mass was 3.2 Pa·s.

The crystal mass was heated to 65°C, and boiling crystallization was continued under vacuum at a pressure of 170 mbar for 4.4 hr until the DS of the crystal mass was 81.5% [3]. A small sample of the mass was centrifuged without washing. This resulted in a crystal cake 2'-FL purity of 97.2% [1], mother liquor 2'-FL purity of 73.5% [1], centrifugation 2'-FL yield of 72% [2], and mother liquor DS of 67.9% [3]. The crystal size of the main crystal population was 30-60 µm [6].

The crystal mass was diluted to a DS of 79.5% [4] by adding deionized water, and then centrifuged with a batch-wise centrifuge having 22.5 cm basket diameter. Here, 693 g of crystal mass was loaded onto the standing centrifuge. Wash water (50 mL) was then added during the acceleration stage at the rotation speed of 2000 rpm. The centrifugation time was continued for 5 min at a rotation speed of 3500 rpm. The 2'-FL centrifugation yield was 54% [2]. The mother liquor 2'-FL purity was 79.5% [1], and the moisture content of the non-dried cake 5.7% [5].

Crystal cake from the centrifugation (274 g) was dried in a heating chamber at 60°C for 2 hr and then cooled in a desiccator. The dried crystals had a 2'-FL purity of 100.0% [1], melting point of 236.8 - 237.5°C [9], color corresponding to 22 ICUMSA [7] and moisture content of 0.3% [3].

Non-dried crystal cake from the centrifugation (30 g) was washed with ethanol, dried and then grinded in a porcelain mill to prepare seed crystals for **Examples 5, 7, 8, 10, 12 and 13.**

### Example 4: 2'-FL Crystallization from Centrifugation Mother Liquor of Example 3

The centrifugation mother liquor from **Example 3**, which had a 2'-FL concentration of 79.5% [1], a DiFL concentration of 17.9% [1], and lactose concentration of <0.1% [1], was evaporated at a temperature of 73°C and pressure of 200 mbar (Rotavapor R-151 evaporator). When the DS was 74.9% [4], crystals formed by spontaneous nucleation (supersaturation 1.55 [8]). The syrup was diluted to a DS of 72.6% [4] and kept at 73°C without vacuum for 1.2 hr. The boiling of the syrup was then continued at a temperature of 73°C and pressure of 175-180 mbar for 4.8 hr. The obtained crystal mass, which had a DS of 83.2% [3], was moved to a glass bottle and kept in a heating chamber at a temperature of 60°C for 69 hr. After 74 hr from the beginning of spontaneous nucleation, centrifugation of a small sample of the mass without washing (Hettich Rotanta 460R centrifuge, 2500 rpm, 10 min) provided a crystalline cake having a 2'-FL purity of 95.7% [1] and a mother liquor having a 2'-FL purity of 51.5% [1]. This corresponds to a 76 % 2'-FL yield [2]. The crystal size of the main crystal population was 40-100 µm [6].

The crystal mass (165 g) was centrifuged with a batch-wise centrifuge without washing (basket diameter 22.5 cm, 3500 rpm, 3 min). The centrifugation 2'-FL yield was 76% [2]. The centrifuged and dried (60°C, 2.4 hr) crystals had a 2'-FL purity of 93.2% [1] with a color of 157 ICUMSA [7] and moisture content of 0.5% [3]. The mother liquor 2'-FL purity was 53.9% [1] with a color of 609 ICUMSA [7].

### Example 5: Use of Fresh Feed Simultaneous with Crystal Precipitation

An aqueous feed syrup, which had a 2'-FL concentration of 87.3% [1], a DiFL concentration of 10.1% [1], and a lactose concentration of 0.2% [1], was evaporated to a DS of 67.7% [4] (Luwa thin film evaporator NL3-210/1600/10). At this point, 1149 g of the syrup was moved to a Rotavapor R-153 evaporator and seeded with 0.83 g of 2'-FL dry seed (crystals from **Example 3**) at a temperature of 61°C (supersaturation of 1.30 [8]). The seeded syrup was boiled at a temperature of 65°C and pressure of 150-165 mbar for 5.1 hr while simultaneously adding fresh feed liquid (DS of 58.3% [4], 1443 g in total) to the crystal mass in small portions (approximately 30 - 120 g) at 5 - 40 min time intervals. Afterward, the DS of the crystal mass was 80.3% [3]. Centrifugation of a small sample of the mass without washing (Hettich Rotanta 460R centrifuge, 2500 rpm, 10 min) provided a crystalline cake having a 2'-FL purity of 96.1% [1] and mother liquor 2'-FL purity of 75.2% [1], which corresponds to a 64% 2'-FL yield [2]. The crystal size of the main crystal population was 30-60 µm [6].

The crystal mass (1332 g) was centrifuged with 75 mL wash water (batchwise centrifuge, basket diameter 22.5 cm, 3500 rpm, 5 min). The 2'-FL centrifugation yield was 53% [2]. The 2'-FL purity of the centrifuged and dried (60°C, 8-17 hr) crystals was 97.8% [1]. The color was 67 ICUMSA [7] and the moisture content was 0.1% [3]. The mother liquor 2'-FL purity was 78.1% [1] and color was 93 ICUMSA [7].

### Example 6: 2'-FL Crystallization from Centrifugation Mother Liquor of Example 5 Using Water Addition Simultaneously with Crystal Precipitation

The centrifugation mother liquor from **Example 5**, which had a 2'-FL concentration of 78.1% [1], a DiFL concentration of 17.6% [1], and a lactose concentration of 0.3% [1], was evaporated at a temperature of 67°C and pressure of 200 mbar (Rotavapor R-151 evaporator). When the DS was 75.5% [4], crystals formed by spontaneous nucleation (supersaturation 1.65 [8]). The syrup was diluted to a DS of 72.6% [4] and kept at 67°C without vacuum for 1 hr. Boiling of the syrup was then continued at a temperature of 65-67°C and pressure of 200 mbar for 5.2 hr while simultaneously adding small portions of deionized water (around 500 g in total) to the mass.

After 6.2 hr from the beginning of spontaneous nucleation, the DS of the crystal mass was 81.8% [3]. Centrifugation of a small sample of the mass without washing (Hettich Rotanta 460R centrifuge, 2500 rpm, 10 min) provided a crystalline cake having a 2'-FL purity of 91.9% [1] and a mother liquor having a 2'-FL purity of 59.0% [1]. This corresponds to a 68% 2'-FL yield [2]. The crystal size of the main crystal population was 30-80 µm [6].

The crystal mass (371 g) was centrifuged with 30 mL wash water (batchwise centrifuge, basket diameter 22.5 cm, 3500 rpm, 5 min). The 2'-FL centrifugation yield was 54% [2]. The 2'-FL purity of the centrifuged and dried (60°C, 2 hr) crystals was 93.4% [1]. The color was 100 ICUMSA [7] and the moisture content was 0.9% [3]. The mother liquor 2'-FL purity was 65.4% [1] and the color was 287 ICUMSA [7].

### Example 7: Illustration of 2'-FL Crystallization

An aqueous feed syrup, which had a 2'-FL concentration of 89.9% [1], a DiFL concentration of 8.5% [1], and a lactose concentration of <0.1% [1], was evaporated to a DS of 65.6% [3] (Luwa thin film evaporator NL3-210/1600/10). The resulting syrup (8141 g) was moved to a Rotavapor R-153 evaporator and seeded with 2.7 g of 2'-FL dry seed (crystals from **Example 3**) at a temperature of 68°C (supersaturation of 1.17 [8]). Mixing was continued at this temperature for 1.2 hr. Afterward, the seeded syrup was evaporated to a DS of 80.4% [3] within 5.3 hr in three steps such that, in each step, the syrup was first boiled under vacuum for 0.9 - 1.2 hr (67 - 72°C and 190 - 200 mbar), followed by mixing at atmospheric pressure for 0.6 - 0.9 hr (67 - 72°C). The resulting crystal mass was moved to a 6 L mixing tank and kept at 69°C overnight (19 hr). Progression of crystallization was monitored by centrifuging (Hettich Rotanta 460R centrifuge, 2500 rpm, 10 min) small samples of the mass without washing. The results are summarized in **Table 1.**

The resulting crystal mass was centrifuged in 3 batches using an amount of wash water equal to 62-82 mL/kg mass DS (batchwise centrifuge, basket diameter 22.5 cm, 3500 rpm, 5 min). The moisture content of the combined, non-dried cake was 4.4% [5]. The 2'-FL purity of the centrifuged and dried (60°C, 2 hr) crystals was 100.0% [1]. The color was 462 ICUMSA [7] and the moisture content was 0.9% [3]. The crystal size of the main crystal population was 30-100 µm [6].

**Table 1**

| **Crystallization in terms of DS [3] or *[4], 2'-FL purity [1] and centrifugation yield [2]** | | | | | | | |
|---|---|---|---|---|---|---|---|
| **time from seeding crystal mass** | | | **mother liquor** | | **crystal cake** | | **centrifuging yield** |
| hr | DS % | 2'-FL % | DS % | 2'-FL % | DS % | 2'-FL % | 2'-FL % |
| 2.7 | 71.1* | 89.9 | 72.0 | 90.6 | | | |
| 4.8 | 76.1* | 89.9 | 70.7 | 86.0 | 95.8 | 98.7 | 34 |
| 6.5 | 80.4 | 89.9 | 69.8 | 80.2 | 95.6 | 98.1 | 59 |
| 25.0 | 80.4 | 89.9 | 66.0 | 75.8 | 95.1 | 97.2 | 71 |

### Example 8: Illustration of 2'-FL Crystallization From Mother Liquor and Equipment Wash from Example 7

The centrifugation mother liquor combined with diluted crystal mass recovered from washing the equipment from **Example 7,** which had a 2'-FL concentration of 87.4% [1], a DiFL concentration of 11.1% [1], and a lactose concentration of <0.1% [1], was evaporated to a DS of 65.1% [4] (Rotavapor R-153 evaporator). The syrup (4812 g) was seeded with 1.75 g of 2'-FL dry seed (crystals from **Example 3**) at a temperature of 65°C (supersaturation of 1.13 [8]). The seeded syrup then was boiled at a temperature of 68-70°C and pressure of 190 - 195 mbar for 6.1 hr. The DS of the resulting crystal mass was 80.8% [3]. Centrifugation of a small sample of the mass without washing (Hettich Rotanta 460R centrifuge, 2500 rpm, 10 min) provided a crystalline cake 2'-FL having a purity of 96.5% [1] and a mother liquor 2'-FL having a purity of 76.7% [1]. This corresponds to a 60% 2'-FL yield [2].

The crystal mass was kept in rotating Rotavapor bottle at a temperature of 68°C overnight (18 hr). After 23 hr from seeding, centrifugation of a small sample of the mass without washing (Hettich Rotanta 460R centrifuge, 2500 rpm, 10 min) provided a crystalline cake having a 2'-FL purity of 97.3% [1] and a mother liquor 2'-FL purity of 69.7% [1]. This corresponds to a 71% 2'-FL yield [2]. The crystal size of the main crystal population was 30-80 µm [6].

The resulting crystal mass was centrifuged in two batches with wash water at an amount equaling 61-75 mL/kg mass DS (batchwise centrifuge, basket diameter 22.5 cm, 3500 rpm, 5 min). The moisture content of the combined, non-dried cake was 3.7% [5]. The 2'-FL purity of the centrifuged and dried (60°C, 2.2 hr) crystals was 97.1% [1]. The color was 389 ICUMSA [7] and the moisture content was 0.5% [3].

### Example 9: Illustration of 2'-FL Crystallization Using Seeding with a Crystalline Mass

The centrifugation mother liquor combined with diluted crystal mass recovered from washing the equipment from **Example 8,** which had a 2'-FL concentration of 82.5% [1], a DiFL concentration of 15.9% [1], and a lactose concentration of <0.1% [1], was evaporated to a DS of 74.7% [4] (Rotavapor R-153 evaporator). The syrup (2455 g) was seeded with 83 g of mother liquor from **Example 8** (which contained small crystals) at a temperature of 67°C (supersaturation of 1.67 [8]). The seeded syrup was boiled at a temperature of 66-68°C and pressure of 190-200 mbar for 4.1 hr. The DS of the resulting crystal mass was 85.5% [3]. Centrifugation of a small sample of the mass without washing (Hettich Rotanta 460R centrifuge, 2500 rpm, 10 min) provided a crystalline cake having a 2'-FL purity of 95.7% [1] and a mother liquor 2'-FL purity of 61.8% [1]. This corresponds to 71% 2'-FL yield [2].

The resulting crystal mass was kept in a rotating Rotavapor bottle at a temperature of 66°C overnight (17 hr). After 21 hr from seeding, centrifugation of a small sample of the mass without washing (Hettich Rotanta 460R centrifuge, 2500 rpm, 10 min) provided a crystalline cake having a 2'-FL purity of 94.0% [1] and a mother liquor 2'-FL purity of 54.0% [1] . This corresponds to a 81% 2'-FL yield [2]. The crystal size of the main crystal population was 80-200 µm [6].

The resulting crystal mass was centrifuged with wash water in an amount equal to 71 mL/kg mass DS (batchwise centrifuge, basket diameter 22.5 cm, 3500 rpm, 5 min). The moisture content of the combined, non-dried cake was 3.4% [5]. The 2'-FL purity of the centrifuged and dried (60°C, 2 hr) crystals was 100.0% [1]. The color was 35 ICUMSA [7] and the moisture content was 0.2% [3].

### Example 10 Illustration of 2'-FL Crystallization from a High Lactose Solution and Using Seeding with a Crystalline Mass

An aqueous feed solution, which had a 2'-FL concentration of 84.5% [1], a DiFL concentration of 7.9% [1], and a lactose concentration of 6.0% [1], was evaporated to a DS of 69.6% [3] (Rotavapor R-153 evaporator). The solution was divided into two parts: one (1394 g) was used for making seeding mass, and the other was used as an actual feed solution (10640 g).

The syrup for seeding mass was seeded with 0.5 g of 2'-FL dry seed (crystals from **Example 3**) at a temperature of 68°C (supersaturation of 1.32 [8]). The seeded syrup was boiled at a temperature of 65-68°C and pressure of 190 mbar for 5 hr. The obtained seeding mass, which had a DS of 84.5% [3], was kept in a rotating Rotavapor bottle at a temperature of 55°C overnight (19 hr).

After 24 hr from the seeding of the seeding mass, the actual feed solution was mixed with the seeding mass at 65°C (supersaturation of 1.32 [8]). The syrup was boiled at a temperature of 68°C and pressure of 190-200 mbar for 9.6 hr. The resulting seeding mass, which had a DS of 80.7% [3], was kept in a rotating Rotavapor bottle at 68°C overnight (16 hr).

After 24 hr from the seeding of actual feed solution, centrifugation of a small sample of the mass without washing (Hettich Rotanta 460R centrifuge, 2500 rpm, 10 min) provided a crystalline cake having a 2'-FL purity of 97.5% [1] and a mother liquor having a 2'-FL purity of 65.3% [1]. This corresponds to a 69% 2'-FL yield [2]. The crystal size of the main crystal population was 100-200 µm [6].

The crystal mass was centrifuged in 6 batches with wash water at an amount equaling 68-83 mL/kg mass DS (batchwise centrifuge, basket diameter 22.5 cm, 3500 rpm, 5 min). The average 2'-FL centrifugation yield over the 6 batches was 56% [2]. The average 2'-FL purity of the centrifuged and dried (18 hr at 40°C and 1 hr at 60°C) crystals was 99.0% [1], the melting point was 234.4-235.9°C [9], the average color was 63 ICUMSA [7], and the moisture content was 0.6% [3]. The mother liquor 2'-FL purity was 71.0% [1] and the color was 850 ICUMSA [7].

### Example 11: Illustration of 2'-FL Crystallization From Mother Liquor and Equipment Wash from Example 10

The centrifugation mother liquor combined with diluted crystal mass recovered from washing the equipment from **Example 10,** which had a 2'-FL concentration of 72.4% [1], a DiFL concentration of 14.0% [1], and a lactose concentration of 10.6% [1], was evaporated to a DS of 70.3% [3] (Rotavapor R-153 evaporator). The syrup (6119 g) was seeded with 1767 g of crystal mass from **Example 10** at a temperature of 65°C (supersaturation of 1.19 [8]). The seeded syrup was boiled at a temperature of 67-71°C and at pressure of 180-190 mbar for 5.4 hr. The DS of the resulting crystal mass was 85.1% [3].

The resulting crystal mass was kept in a rotating Rotavapor bottle at a temperature of 68°C overnight (18 hr). After 24 hr from seeding, centrifugation of a small sample of the mass without washing (Hettich Rotanta 460R centrifuge, 2500 rpm, 10 min) provided a crystalline cake having a 2'-FL purity of 94.1% [1] and a mother liquor 2'-FL purity of 46.5% [1]. This corresponds to 76% 2'-FL yield [2]. The crystal size of the main crystal population was 100-200 µm [6].

The crystal mass was centrifuged in 4 batches with wash water in an amount equal to 70-87 mL/kg mass DS (batchwise centrifuge, basket diameter 22.5 cm, 3500 rpm, 10 min). The average 2'-FL centrifugation yield was 55% [2]. The average 2'-FL purity of the centrifuged and dried (18 hr at 40°C and 1 hr at 60°C) crystals was 97.9% [1]. The color was 204 ICUMSA [7] and the moisture content was 0.8% [3]. The mother liquor 2'-FL purity was 59.4% [1] and the color was 1409 ICUMSA [7].

### Example 12: Illustration of 2'-FL Recrystallization

2'-FL crystals (1842 g) from **Example 11,** which had a 2'-FL concentration of 97.9% [1], a DiFL concentration of 0.9% [1], and a lactose concentration of 1.3% [1], were dissolved in deionized water. The resulting solution was evaporated to a DS of 65.1% [3] (Rotavapor R-153 evaporator), and seeded with 0.88 g of 2'-FL dry seed (crystals from **Example 3**) at a temperature of 60°C (supersaturation of 1.30 [8]). The seeded syrup was boiled at a temperature of 64-65°C and pressure of 180 mbar for 4.7 hr. The DS of the resulting crystal mass was 77.8% [3].

The resulting crystal mass was kept in a rotating Rotavapor bottle at a temperature of 64°C overnight (19 hr). After 24 hr from seeding, centrifugation of a small sample of the mass without washing (Hettich Rotanta 460R centrifuge, 2500 rpm, 10 min) provided a crystalline cake having a 2'-FL purity of 99.7% [1] and a mother liquor 2'-FL purity of 95.5% [1]. This corresponds to 55% 2'-FL yield [2]. The crystal size of the main crystal population was 30-100 µm [6].

The crystal mass was centrifuged with wash water in an amount equal to 71 mL/kg mass DS (batchwise centrifuge, basket diameter 22.5 cm, 3500 rpm, 5 min). The moisture content of the combined, non-dried cake was 5.8% [5]. The 2'-FL purity of the centrifuged and dried (21 hr at 40°C and 1 hr at 60°C) crystals was 100.0% [1]. The color was 142 ICUMSA [7] and the moisture content was 0.1% [3].

### Example 13: Illustration of 2'-FL Crystallization

An aqueous feed syrup, which had a 2'-FL concentration of 89.6% [1], a DiFL concentration of 8.8% [1], and a lactose concentration of <0.1% [1], was evaporated to a DS of 67.5% [3] (Rotavapor R-153 evaporator). The resulting syrup (1343 g) was seeded with 0.44 g of 2'-FL dry seed (crystals from **Example 3**) at a temperature of 61°C (supersaturation of 1.32 [8]). The seeded syrup was boiled at a temperature of 64-65°C and pressure of 180 mbar for 5.5 hr. The DS of the resulting crystal mass was 81.4% [3].

The resulting crystal mass was kept in a rotating Rotavapor bottle at a temperature of 66°C overnight (23 hr). After 26 hr from seeding, centrifugation of a small sample of the mass without washing (Hettich Rotanta 460R centrifuge, 2500 rpm, 10 min) provided a crystalline cake having a 2'-FL purity of 98.1% [1] and a mother liquor 2'-FL purity of 76.1% [1]. This corresponds to 67% 2'-FL yield [2]. The crystal size of the main crystal population was 30-100 µm [6].

The crystal mass was centrifuged with wash water in an amount equal to 75 mL/kg mass DS (batchwise centrifuge, basket diameter 22.5 cm, 3500 rpm, 5 min). The moisture content of the combined, non-dried cake was 4.6% [5]. The 2'-FL purity of the centrifuged and dried (19 hr at 40°C and 1 hr at 60°C) crystals was 100.0% [1]. The melting point was 233.2 - 234.1°C [9], the color was 47 ICUMSA [7], and the moisture content was 0.1% [3].

### Example 14: Illustration of 2'-FL Crystallization from a Low 2'-FL Solution

An aqueous feed solution, which had a 2'-FL concentration of 60.5% [10], a DiFL concentration of 20.7% [10], and a lactose concentration of 15.1% [10], was evaporated to a DS of about 75%. The syrup was then seeded with 2'-FL dry seed crystals in an amount equal to about 0.18 g/kg syrup DS at a temperature of about 65°C (supersaturation of about 1.3 [8]). Mixing was continued at this temperature for about 0.5 hr. Afterward, the seeded syrup was evaporated to a DS of about 81.5% within about 20 hr at a temperature of 65-70°C.

The resulting crystal mass was kept mixing at 75 °C for about 6 hr, and then centrifuged with wash water in an amount equal to about 60 mL/kg mass DS. The average 2'-FL centrifugation yield was 17.2%, and the average 2'-FL purity of the centrifuged crystals was 97.4% [10].

### Example 15: Solubility of 2'-FL in Water

Dried 2'-FL crystals from **Example 9** and deionized water were weighed in a 100 mL laboratory bottle. The container was tempered to a specific temperature (25, 40, 50, 60, or 70°C) using a thermostat water bath. The contents were stirred continuously with a magnetic stirrer for at least 16 hr to reach solubility equilibrium. 2'-FL crystals were added during the experiment such that the crystal content at the equilibrium was around 10-20 vol%.

After the system had reached equilibrium, mother liquor was separated from the crystals by settling. The 2'-FL concentration of the mother liquor was determined by Karl Fischer titration. The results are summarized in **Table 2.**

**Table 2**

| **Solubility of 2'-FL in water** | |
|---|---|
| temperature | solubility |
| °C | g 2'-FL / 100 g water |
| 25 | 141 |
| 40 | 133 |
| 50 | 137 |
| 60 | 139 |
| 70 | 149 |

### Example 16: Preparation of Amorphous 2'-FL from Crystalline 2'-FL

A solution of crystalline 2'-FL dissolved in water was spray dried to obtain amorphous 2'-FL in powder form. The 2'-FL solution had the characteristics shown in **Table 3.**

**Table 3**

| **Parameter** | **Value** |
|---|---|
| Total amount | 826 kg |
| Brix | 47.48 %Brix |
| Calculated % solids | 45.5% |
| pH | 8.15 |
| Conductivity | 2 µS/cm |
| Protein concentration | 2.69 mg/L |
| Temperature | ~30°C |

An SPX Anhydro CSD Type 70 (vol. = ~43 m³) (Soeborg, Denmark) co-current spray dryer equipped with an atomizer wheel was used to spray dry the 2'-FL solution. The 2'-FL solution was fed into the spray dryer at an initial rate of 79 kg/h, which was increased to 115 kg/h over the course of the first 3 hours of spray drying. The spray dryer settings were as shown in **Table 4.**

**Table 4**

| **Parameter** | **Value** |
|---|---|
| Atomizer speed | 25,136 rpm |
| Air inlet flow | 3,000 m³/h |
| Air inlet temperature | 135°C |
| Air outlet temperature | 104°C |

The drying process lasted for 457 minutes.

The dried 2'-FL powder obtained had the characteristics shown in **Table 5.**

**Table 5**

| **Parameter** | **Value** |
|---|---|
| Total amount | 340.1 kg |
| Product yield | 90.5% |
| Loose bulk density | 601 g/L |
| 100x tapped bulk density | 772 g/L |
| 625x tapped bulk density | 832 g/L |
| Moisture content (by weight) | 2.12-2.21% |

Bulk density of the 70 g of powder was measured using a Jolting Stampf Volumeter (STAV 203, J. Engelsmann AG), a 250 mL measuring cylinder and a technical weighing scale. Moisture content of the powder was measured using Karl-Fischer titration.

## Claims

1. A process for making crystalline 2'-fucosyllactose from an aqueous starting solution comprising 2'-fucosyllactose and at least one other carbohydrate, wherein:
the process comprises:
concentrating the starting solution to a supersaturated state with respect to 2'-fucosyllactose, and
precipitating a 2'-fucosyllactose crystal from the supersaturated solution while subjecting the supersaturated solution to a temperature of greater than 60°C; and
the supersaturated solution comprises no greater than 1% by weight organic solvent during the precipitation of the 2'-fucosyllactose crystal, optionally wherein the supersaturated solution comprises less than 0.1% by weight organic solvent during precipitation of the 2'-fucosyllactose crystal,
wherein the term supersaturated corresponds to a dimensionless ratio of the measured 2'-fucosyllactose content to the solubility of 2'-fucosyllactose, the ratio being calculated from the following equation $s = \frac{2 ′ - fucosyllactose content in solution}{solubility of 2 ′ - fucosyllactose at the same temperature}$ where s is greater than 1; and
wherein,
(A) the at least one other carbohydrate comprises lactose, and
the starting solution has a dry solids content with a lactose concentration of at least 0.1%, wherein the percentage corresponds to a normalized peak area concentration obtained using high performance liquid chromatography; or
(B) the at least one other carbohydrate comprises difucosyllactose, and
the starting solution has a dry solids content with a difucosyllactose concentration of at least 1%, wherein the percentage corresponds to a normalized peak area concentration obtained using high performance liquid chromatography.

2. The process according to claim 1, wherein the organic solvent is an organic acid solvent.

3. The process according to claim 2, wherein the organic acid solvent is acetic acid.

4. The process according to any one of the preceding claims, wherein the starting solution has a dry solids content with a 2'-fucosyllactose concentration of less than 98%, wherein the percentage corresponds to a normalized peak area concentration obtained using high performance liquid chromatography.

5. The process according to claim 4, wherein the starting solution has a dry solids content with a 2'-fucosyllactose concentration of from 70 to 95%, wherein the percentage corresponds to a normalized peak area concentration obtained using high performance liquid chromatography.

6. The process according to any one of the preceding claims, wherein no organic solvent is added to the supersaturated solution while the supersaturated solution is being subjected to a temperature of greater than 60°C.

7. The process according to any one of the preceding claims, wherein the starting solution is derived from a fermentation.

8. The process according to any one of the preceding claims, wherein the starting solution comprises less than 0.1% by weight organic solvent.

9. The process according to any one of the preceding claims, wherein no organic solvent is added to the starting solution before or during concentration of the starting solution to a supersaturated state.

10. The process according to any one of the preceding claims, wherein the crystalline 2'-fucosyllactose has a melting point of from about 230 to about 239°C as determined with a 1°C/min heating rating using the European Pharmacopoeia capillary melting point method.

11. The process according to any one of the preceding claims, wherein the crystalline 2'-fucosyllactose exhibits an X-ray powder diffraction reflection, based on a measurement using CuKa radiation, at 16.98±0.20, 13.65±0.20 and 18.32±0.20 2Θ angles.

12. The process according to any one of the preceding claims, wherein 2'-fucosyllactose crystal is precipitated from the supersaturated solution while subjecting the supersaturated solution to a temperature of greater than 60°C and no greater than 80°C.

13. A process for making amorphous 2'-fucosyllactose, wherein the process comprises:
making crystalline 2'-fucosyllactose according to the process of any one of the preceding claims,
dissolving the crystalline 2'-fucosyllactose in a solvent to form a purified 2'-fucosyllactose solution, and
precipitating amorphous 2'-fucosyllactose from the purified 2'-fucosyllactose solution, wherein the precipitation of amorphous 2'-fucosyllactose from the purified 2'-fucosyllactose solution comprises spray drying.

14. A process for making a food, dietary supplement or medicine, wherein the process comprises:
making crystalline 2'-fucosyllactose according to the process of any one of claims 1 to 12, and
either:
mixing the crystalline 2'-fucosyllactose with one or more ingredients suitable for the food, dietary supplement or medicine; or
dissolving the crystalline 2'-fucosyllactose in a solvent, and
mixing the dissolved 2'-fucosyllactose with one or more ingredients suitable for the food, dietary supplement or medicine.

15. A process for making a food, dietary supplement or medicine, wherein the process comprises:
making amorphous 2'-fucosyllactose according to the process of claim 13, and
either:
mixing the amorphous 2'-fucosyllactose with one or more ingredients suitable for the food, dietary supplement or medicine; or
dissolving the amorphous 2'-fucosyllactose in a solvent, and
mixing the dissolved 2'-fucosyllactose with one or more ingredients suitable for the food, dietary supplement or medicine.

## Patentansprüche

1. Verfahren zur Herstellung von kristalliner 2'-Fucosyllactose aus einer wässrigen Ausgangslösung, die 2'-Fucosyllactose und mindestens ein anderes Kohlenhydrat umfasst, wobei:
das Verfahren Folgendes umfasst:
das Aufkonzentrieren der Ausgangslösung in einen übersättigten Zustand in Bezug auf 2'-Fucosyllactose und das Ausfällen eines 2'-Fucosyllactosekristalls aus der übersättigten Lösung, während die übersättigte Lösung einer Temperatur von mehr als 60 °C ausgesetzt wird; und die übersättigte Lösung während der Ausfällung des 2'-Fucosyllactosekristalls nicht mehr als 1 Gew.-% organisches Lösungsmittel umfasst, wobei die übersättigte Lösung während der Ausfällung des 2'-Fucosyllactosekristalls gegebenenfalls weniger als 0,1 Gew.-% organisches Lösungsmittel umfasst,
wobei der Begriff "übersättigt" einem dimensionslosen Verhältnis des gemessenen 2'-Fucosyllactosegehalts zur Löslichkeit von 2'-Fucosyllactose entspricht, wobei das Verhältnis aus der folgenden Gleichung berechnet wird $s = \frac{2 ′ - Fucosyllactosegehalt in Lösung}{L ö slichkeit von 2 ′ - Fucosyllactose bei der gleichen Temperatur}$
wobei s größer als 1 ist und
wobei
(A) das mindestens eine andere Kohlenhydrat Lactose umfasst und
die Ausgangslösung einen Trockenfeststoffgehalt mit einer Lactosekonzentration von mindestens 0,1 % aufweist, wobei der Prozentsatz einer mittels Hochleistungsflüssigkeitschromatographie erhaltenen normalisierten Peakflächenkonzentration entspricht, oder
(B) das mindestens eine andere Kohlenhydrat Difucosyllactose umfasst und
die Ausgangslösung einen Trockenfeststoffgehalt mit einer Difucosyllactosekonzentration von mindestens 1 % aufweist, wobei der Prozentsatz einer mittels Hochleistungsflüssigkeitschromatographie erhaltenen normalisierten Peakflächenkonzentration entspricht.

2. Verfahren nach Anspruch 1, wobei es sich bei dem organischen Lösungsmittel um ein organisches saures Lösungsmittel handelt.

3. Verfahren nach Anspruch 2, wobei es sich bei dem organischen sauren Lösungsmittel um Essigsäure handelt.

4. Verfahren nach einem der vorhergehenden Ansprüche, wobei die Ausgangslösung einen Trockenfeststoffgehalt mit einer 2'-Fucosyllactosekonzentration von weniger als 98 % aufweist, wobei der Prozentsatz einer mittels Hochleistungsflüssigkeitschromatographie erhaltenen normalisierten Peakflächenkonzentration entspricht.

5. Verfahren nach Anspruch 4, wobei die Ausgangslösung einen Trockenfeststoffgehalt mit einer 2'-Fucosyllactosekonzentration von 70 bis 95 % aufweist, wobei der Prozentsatz einer mittels Hochleistungsflüssigkeitschromatographie erhaltenen normalisierten Peakflächenkonzentration entspricht.

6. Verfahren nach einem der vorhergehenden Ansprüche, wobei der übersättigten Lösung kein organisches Lösungsmittel zugesetzt wird, während die übersättigte Lösung einer Temperatur von mehr als 60 °C ausgesetzt wird

7. Verfahren nach einem der vorhergehenden Ansprüche, wobei die Ausgangslösung aus einer Fermentation stammt.

8. Verfahren nach einem der vorhergehenden Ansprüche, wobei die Ausgangslösung weniger als 0,1 Gew.-% organisches Lösungsmittel umfasst.

9. Verfahren nach einem der vorhergehenden Ansprüche, wobei der Ausgangslösung vor oder während des Aufkonzentrierens der Ausgangslösung in einen übersättigten Zustand kein organisches Lösungsmittel zugesetzt wird.

10. Verfahren nach einem der vorhergehenden Ansprüche, wobei die kristalline 2'-Fucosyllactose einen mit einer Aufheizrate von 1 °C/min unter Anwendung des European Pharmacopoeia Kapillarschmelzpunktverfahrens bestimmten Schmelzpunkt von etwa 230 bis etwa 239 °C aufweist.

11. Verfahren nach einem der vorhergehenden Ansprüche, wobei die kristalline 2'-Fucosyllactose eine Röntgenpulverbeugungsreflexion, basierend auf einer Messung unter Verwendung von CuKa-Strahlung, bei 16,98±0,20, 13,65±0,20 und 18,32±0,20 2Θ-Winkeln zeigt.

12. Verfahren nach einem der vorhergehenden Ansprüche, wobei 2'-Fucosyllactosekristall aus der übersättigten Lösung ausgefällt wird, während die übersättigte Lösung einer Temperatur von mehr als 60 °C und nicht mehr als 80 °C ausgesetzt wird.

13. Verfahren zur Herstellung von amorpher 2'-Fucosyllactose, wobei das Verfahren Folgendes umfasst:
das Herstellen von kristalliner 2'-Fucosyllactose gemäß dem Verfahren nach einem der vorhergehenden Ansprüche, das Lösen der kristallinen 2'-Fucosyllactose in einem Lösungsmittel unter Bildung einer Lösung von aufgereinigter 2'-Fucosyllactose und
das Ausfällen von amorpher 2'-Fucosyllactose aus der Lösung von aufgereinigter 2'-Fucosyllactose, wobei die Ausfällung von amorpher 2'-Fucosyllactose aus der Lösung von aufgereinigter 2'-Fucosyllactose eine Sprühtrocknung umfasst.

14. Verfahren zur Herstellung eines Nahrungsmittels, Nahrungsergänzungsmittels oder Arzneimittels, wobei das Verfahren Folgendes umfasst:
das Herstellen von kristalliner 2'-Fucosyllactose gemäß dem Verfahren nach einem der Ansprüche 1 bis 12 und
entweder:
das Mischen der kristallinen 2'-Fucosyllactose mit einer oder mehreren für das Lebensmittel, das Nahrungsergänzungsmittel oder das Arzneimittel geeigneten Zutaten oder
das Lösen der kristallinen 2'-Fucosyllactose in einem Lösungsmittel und
das Mischen der gelösten 2'-Fucosyllactose mit einer oder mehreren für das Lebensmittel, das Nahrungsergänzungsmittel oder das Arzneimittel geeigneten Zutaten.

15. Verfahren zur Herstellung eines Nahrungsmittels, Nahrungsergänzungsmittels oder Arzneimittels, wobei das Verfahren Folgendes umfasst:
das Herstellen von amorpher 2'-Fucosyllactose gemäß dem Verfahren nach Anspruch 13 und entweder:
das Mischen der amorphen 2'-Fucosyllactose mit einer oder mehreren für das Lebensmittel, das Nahrungsergänzungsmittel oder das Arzneimittel geeigneten Zutaten oder
das Lösen der amorphen 2'-Fucosyllactose in einem Lösungsmittel und
das Mischen der gelösten 2'-Fucosyllactose mit einer oder mehreren für das Lebensmittel, das Nahrungsergänzungsmittel oder das Arzneimittel geeigneten Zutaten.

## Revendications

1. Procédé de préparation de 2'-fucosyllactose cristallin à partir d'une solution aqueuse de départ comprenant du 2'-fucosyllactose et au moins un autre glucide, dans lequel :
le procédé comprend :
concentrer la solution de départ jusqu'à un état sursaturé en 2'-fucosyllactose, et
précipiter un cristal de 2'-fucosyllactose de la solution sursaturée tout en soumettant la solution sursaturée à une température supérieure à 60 °C ; et
la solution sursaturée ne comprend pas plus de 1 % en poids de solvant organique lors de la précipitation du cristal de 2'-fucosyllactose, éventuellement la solution sursaturée comprenant moins de 0,1 % en poids de solvant organique lors de la précipitation du cristal de 2'-fucosyllactose,
dans lequel le terme sursaturé correspond à un rapport sans dimension de la teneur en 2'-fucosyllactose mesurée sur la solubilité du 2'-fucosyllactose, le rapport étant calculé à partir de l'équation suivante $s = \frac{teneur en 2 ′ - fucosyllactose en solution}{solubilit é de 2 ′ - fucosyllactose à la même température}$
où s est supérieur à 1 ; et
dans lequel
(A)l'au moins un autre glucide comprend du lactose, et la solution de départ a une teneur en solides secs avec une concentration en lactose d'au moins 0,1 %, le pourcentage correspondant à une concentration de surface de pic normalisée obtenue en utilisant une chromatographie liquide haute performance ; ou
(B)l'au moins un autre glucide comprend du difucosyllactose, et
la solution de départ présente une teneur en solides secs avec une concentration de difucosyllactose d'au moins 1 %, le pourcentage correspondant à une concentration de surface de pic normalisée obtenue en utilisant une chromatographie liquide haute performance.

2. Procédé selon la revendication 1, dans lequel le solvant organique est un solvant d'acide organique.

3. Procédé selon la revendication 2, dans lequel le solvant acide organique est l'acide acétique.

4. Procédé selon l'une quelconque des revendications précédentes, dans lequel la solution de départ a une teneur en solides secs avec une concentration en 2'-fucosyllactose inférieure à 98 %, dans lequel le pourcentage correspond à une concentration de surface de pic normalisée obtenue en utilisant une chromatographie liquide haute performance.

5. Procédé selon la revendication 4, dans lequel la solution de départ a une teneur en solides secs avec une concentration en 2'-fucosyllactose de 70 à 95 %, dans lequel le pourcentage correspond à une concentration de surface de pic normalisée obtenue en utilisant une chromatographie liquide haute performance.

6. Procédé selon l'une quelconque des revendications précédentes, dans lequel aucun solvant organique n'est ajouté à la solution sursaturée alors que la solution sursaturée est soumise à une température supérieure à 60 °C.

7. Procédé selon l'une quelconque des revendications précédentes, dans lequel la solution de départ est dérivée d'une fermentation.

8. Procédé selon l'une quelconque des revendications précédentes, dans lequel la solution de départ comprend moins de 0,1 % en poids de solvant organique.

9. Procédé selon l'une quelconque des revendications précédentes, dans lequel aucun solvant organique n'est ajouté à la solution de départ avant ou pendant la concentration de la solution de départ jusqu'à un état sursaturé.

10. Procédé selon l'une quelconque des revendications précédentes, dans lequel le 2'-fucosyllactose cristallin a un point de fusion d'environ 230 à environ 239 °C tel que déterminé avec une vitesse de chauffage 1 °C/min en utilisant le procédé du point de fusion capillaire de la Pharmacopée européenne.

11. Procédé selon l'une quelconque des revendications précédentes, dans lequel le 2'-fucosyllactose cristallin présente une réflexion de diffraction des rayons X sur poudre, basée sur une mesure utilisant un rayonnement CuKa, à des angles de 16,98 ± 0,20, 13,65 ± 0,20 et 18,32 ± 0,20 2Θ.

12. Procédé selon l'une quelconque des revendications précédentes, dans lequel le cristal de 2'-fucosyllactose est précipité à partir de la solution sursaturée tout en soumettant la solution sursaturée à une température supérieure à 60 °C et non supérieure à 80 °C.

13. Procédé de préparation de 2'-fucosyllactose amorphe, le procédé comprenant :
la préparation de 2'-fucosyllactose cristallin selon le procédé de l'une quelconque des revendications précédentes,
la dissolution du 2'-fucosyllactose cristallin dans un solvant pour former une solution de 2'-fucosyllactose purifié, et
la précipitation de 2'-fucosyllactose amorphe à partir de la solution de 2'-fucosyllactose purifié, dans lequel la précipitation de 2'-fucosyllactose amorphe à partir de la solution de 2'-fucosyllactose purifié comprend un séchage par pulvérisation.

14. Procédé pour la préparation d'un aliment, d'un complément alimentaire ou d'un médicament, dans lequel le procédé comprend :
préparation de 2'-fucosyllactose cristallin selon le procédé de l'une quelconque des revendications 1 à 12, et
soit :
mélange du 2'-fucosyllactose cristallin avec un ou plusieurs ingrédients appropriés pour l'aliment, le complément alimentaire ou le médicament ; soit
dissolution du 2'-fucosyllactose cristallin dans un solvant, et
mélange du 2'-fucosyllactose dissous avec un ou plusieurs ingrédients appropriés pour l'aliment, le complément alimentaire ou le médicament.

15. Procédé pour la préparation d'un aliment, d'un complément alimentaire ou d'un médicament, dans lequel le procédé comprend :
préparation du 2'-fucosyllactose amorphe selon le procédé de la revendication 13, et soit :
mélange du 2'-fucosyllactose amorphe avec un ou plusieurs ingrédients appropriés pour l'aliment, le complément alimentaire ou le médicament ; soit
dissolution du 2'-fucosyllactose amorphe dans un solvant, et
mélange du 2'-fucosyllactose dissous avec un ou plusieurs ingrédients appropriés pour l'aliment, le complément alimentaire ou le médicament.
